# EUROPÄISCHE PATENTSCHRIFT

(11) **EP 3 286 173 B1**
(45) Veröffentlichungstag und Bekanntmachung des Hinweises auf die Patenterteilung: **03.07.2019**
(21) Anmeldenummer: 16716602.4
(22) Anmeldetag: 15.04.2016
(51) Int. Cl.: C07D 317/40, C07D 407/06

(54) **CYCLISCHE CARBONATE**
CYCLIC CARBONATES
CARBONATES CYCLIQUES

(30) Priorität: 23.04.2015 EP 15164849
(43) Veröffentlichungstag der Anmeldung: 28.02.2018
(73) Patentinhaber: BASF SE, 67056 Ludwigshafen am Rhein (DE)
(72) Erfinder: MORMUL, Verena, 68161 Mannheim (DE); KLOPSCH, Rainer, 67551 Worms (DE); YU, Miran, 67547 Worms (DE); SCHERR, Guenter, 67065 Ludwigshafen (DE); GHISLIERI, Diego, 64646 Heppenheim (DE)
(74) Vertreter: BASF IP Association
(86) Internationale Anmeldenummer: PCT/EP2016/058418
(87) Internationale Veröffentlichungsnummer: WO 2016/169858

(56) Entgegenhaltungen:
- WO-A1-03/048215
- WO-A1-2011/157671
- WO-A1-2013/144299

## Beschreibung

Die Erfindung betrifft Verbindungen der Formel 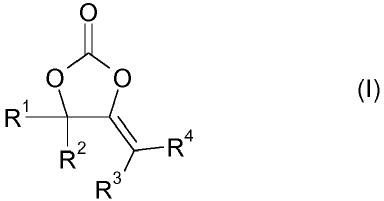 worin
R¹ für einen organischen Rest mit einer (Meth)acrylgruppe steht,
R², R³ und R⁴ unabhängig voneinander für ein H-Atom oder eine C1- bis C10-Alkylgruppe stehen und ihre Verwendung in Epoxidharz-zusammensetzungen

Verbindungen mit einer cyclischen Carbonatgruppe und ihre Verwendung in Epoxidharzzusammensetzungen sind in WO 2011/157671 und WO 2013/050311 beschrieben.

WO 2013/050311 offenbart Verbindungen mit einer cyclischen Carbonatgruppe, welche durch eine elektronenziehende Gruppe, insbesondere eine Carboxylgruppe substituiert sind.

WO 2011/157671 offenbart eine cyclische Carbonatverbindung mit einer Doppelbindung direkt am Ringsystem, welche auch als exo-Vinylencarbonat bezeichnet wird. Das exo-Vinylencarbonat enthält ebenfalls Reste R¹ bis R⁴, siehe Verbindung I der WO 2011/157671. Keiner der Reste R¹ bis R⁴ kann jedoch eine (Meth)acrylgruppe enthalten. Konkret werden in WO 2011/157671 nur cyclische Carbonatverbindungen genannt und eingesetzt, welche nicht polymerisierbar sind.

Verbindungen mit einer cyclischen Carbonatgruppe und mit einer polymerisierbaren Doppelbindung sind aus WO 2013/144299 bekannt. Die polymerisierbare Doppelbindung ist hier über einen Spacer an die exo-Vinylengruppe des exo-Vinylencarbonat der WO 2011/157671 gebunden. Die cyclischen Carbonatverbindungen der WO 2013/144299 werden polymerisiert und finden als Polymere beziehungsweise Copolymere verschiedenste Verwendungen.

Epoxidharze sind üblicherweise Verbindungen mit im Mittel mehr als einer Epoxidgruppe pro Molekül. Diese können durch Umsetzung mit geeigneten Härtern in Polymere überführt werden. Aufgrund ihrer guten anwendungstechnischen Eigenschaften, wie hohe Schlagzähigkeit, hohe Abriebfestigkeit, gute Chemikalienbeständigkeit, hohe Beständigkeit gegenüber Laugen, Säuren, Ölen und organischen Lösungsmitteln finden Epoxidharze vielfältige Verwendungen, z.B. als Beschichtungsmassen oder Klebstoff.

Bei der Verwendung von Epoxidharzen ist eine geringe Viskosität vorteilhaft. Geringe Viskositäten erlauben niedrige Temperaturen bei der Verwendung, erleichtern generell die Verarbeitungsverfahren und ergeben im Allgemeinen gute Ergebnisse, insbesondere gleichmäßige Beschichtungen und gleichmäßige Formkörper.

Daher werden den ungehärteten Epoxidharzen häufig Verdünnungsmittel zugesetzt, welche die Viskosität des Harzes auf den für die Anwendung gewünschten Wert verringern. Geeignete Verdünnungsmittel sind insbesondere Reaktivverdünner. Reaktivverdünner sind Lösungsmittel, welche funktionelle Gruppen aufweisen, welche mit den Epoxidgruppen des Harzes und/oder den funktionellen Gruppen des Härters unter Ausbildung kovalenter Bindungen reagieren.

Die oben genannten WO 2013/050311 und WO 2011/157671 betreffen die Verwendung der offenbarten cyclischen Carbonate als Reaktivverdünner in derartigen Epoxidharzen.

Grundsätzlich besteht gegenüber dem Stand der Technik die Aufgabe, alternative Verdünnungsmittel für Epoxidharz-zusammensetzungen zu finden, welche die Viskosität herabsetzen und die anwendungstechnischen Eigenschaften, insbesondere die Reaktivität, nicht beeinträchtigen oder sogar weiter verbessern. Von besonderem Interesse sind dabei Verdünnungsmittel, welche durch ein effektives und kostengünstiges Herstellverfahren erhalten werden können.

Aufgabe der vorliegenden Erfindung war daher, derartige Verdünnungsmittel zur Verfügung zu stellen.

Demgemäß wurden die eingangs definierten Verbindungen gefunden. Gefunden wurde auch ein Herstellungsverfahren für diese Verbindungen.

Zu den Verbindungen der Formel (I)
In Formel (I) steht R¹ für einen organischen Rest mit einer (Meth)acrylgruppe.
R², R³ und R⁴ stehen unabhängig voneinander für ein H-Atom oder eine C1- bis C10-Alkylgruppe. Verbindungen der Formel I werden im Nachfolgenden auch als ExoVC-(meth)acrylate bezeichnet.

Vorzugsweise steht R¹ für einen organischen Rest mit insgesamt maximal 24 C-Atomen, insbesondere maximal 18 C-Atomen und besonders bevorzugt maximal 14 C-Atomen. R¹ kann neben Sauerstoff weitere Heteroatome wie Stickstoff oder Schwefel enthalten.

In einer besonderen Ausführungsform besteht R¹ ausschließlich aus Kohlenstoff, Wasserstoff und Sauerstoff und enthält daher außer Sauerstoffatomen keine weiteren Heteroatome. Sauerstoffatome finden sich in der Acrylgruppe bzw. Methacrylgruppe, kurz (Meth)acrylgruppe und gegebenenfalls in weiteren funktionellen Gruppen wie Ethergruppen, Hydroxygruppen oder Carbonylgruppen. Insbesondere enthält R¹ Sauerstoff in der (Meth)acrylgruppe und darüberhinaus nur noch in gegebenenfalls vorhandenen Ethergruppen.

Besonders bevorzugt handelt es sich bei R¹ um eine der nachstehenden Gruppen der Formel (II), (III) oder (IV).

In Formel (II) 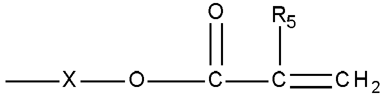 steht X für eine Bindung oder eine Alkylengruppe mit 1 bis 18 C-Atomen und R⁵ steht für ein H-Atom oder eine Methylgruppe.

Vorzugsweise steht X für eine Alkylengruppe mit 1 bis 18 C-Atomen.

Bei der Alkylengruppe kann es sich um eine lineare oder verzweigte Alkylengruppe handeln.

Besonders bevorzugt handelt es sich um eine Alkylengruppe mit 1 bis 10 C-Atomen. Insbesondere handelt es sich um eine lineare C1- bis C10-Alkylengruppe. In einer besonderen Ausführungsform handelt es sich um eine lineare C2- bis C8-Alkylengruppe. Insbesondere handelt es sich um eine lineare C2- bis C6 Alkylengruppe, z.B. eine Ethylen-, n-Propylen oder n- Butylengruppe; ganz besonders bevorzugt ist X eine n-Propylengruppe.

In Formel (III) 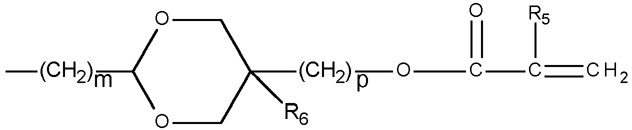 stehen m und p unabhängig voneinander für 0 oder eine ganze Zahl von 1 bis 10, R⁵ hat die obige Bedeutung und R⁶ steht für ein H-Atom oder eine C1- bis C10-Alkylgruppe.

Vorzugsweise steht m für eine ganze Zahl von 1 bis 10, insbesondere für eine ganze Zahl von 1 bis 6, ganz besonders bevorzugt steht m für 1.

Vorzugsweise steht p für 0 oder eine ganze Zahl von 1 bis 6, insbesondere steht p für eine ganze Zahl von 1 bis 6, ganz besonders bevorzugt steht p für 1.

In einer besonderen Ausführungsform stehen m und p unabhängig voneinander für eine ganze Zahl von 1 bis 6. In einer ganz besonderen Ausführungsform stehen sowohl m als auch p für 1.

R⁶ steht vorzugsweise für ein H-Atom oder eine C1- bis C6-Alkylgruppe. In einer besonderen Ausführungsform steht R⁶ für eine C1 bis C6- Alkylgruppen, zum Beispiel eine Methylgruppe oder Ethylgruppe, insbesondere eine Ethylgruppe.

In Formel (IV) 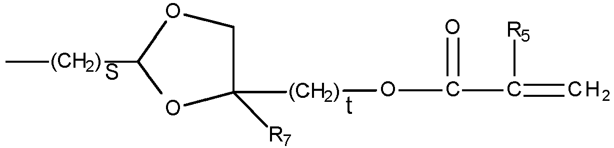 stehen s und t unabhängig voneinander für 0 oder eine ganze Zahl von 1 bis 10, R⁵ hat die obige Bedeutung und R⁷ steht für ein H-Atom oder eine C1- bis C10-Alkylgruppe.

Vorzugsweise stehen s und t unabhängig voneinander für eine ganze Zahl von 1 bis 10, insbesondere eine ganze Zahl von 1 bis 6. In einer besonderen Ausführungsform stehen sowohl s als auch t für 1.
R⁷ steht vorzugsweise für ein H-Atom oder eine C1- bis C6-Alkylgruppe. In einer besonderen Ausführungsform steht R⁷ für ein H-Atom.

In den Verbindungen der Formel I steht R² vorzugsweise für ein H-Atom oder eine C1- bis C6-Alkylgruppe. Insbesondere steht R² für eine C1- bis C6-Alkylgruppe, besonderes bevorzugt für eine Methylgruppe.

In den Verbindungen der Formel I stehen R³ und R⁴ unabhängig voneinander für ein H-Atom oder eine C1- bis C10-Alkylgruppe. Bei der Alkylgruppe handelt es sich insbesondere um eine C1- bis C6-Alkylgruppe, bevorzugt eine C1 bis C3-Alkylgruppe und in einer besonderen Ausführungsform um eine Methylgruppe.

Vorzugsweise steht mindestens einer der Reste R³ und R⁴ für ein H Atom.

Besonderes bevorzugt stehen sowohl R³ als auch R⁴ für ein H-Atom oder einer der R³ und R⁴ für ein H-Atom und der andere für eine Alkylgruppe, insbesondere eine Methyl- oder Ethylgruppe.

Ganz besonderes bevorzugt stehen sowohl R³ als auch R⁴ für ein H-Atom.

R5 steht vorzugsweise in allen Verbindungen der Formel I für ein H-Atom, es handelt sich daher bei Verbindungen der Formel I vorzugsweise um Acrylverbindungen.

Besonders bevorzugte Verbindungen der Formel I sind solche in denen es sich bei R¹ um eine Gruppe der Formel (II) oder (III), insbesondere um eine Gruppe der Formel (II) handelt.

Als Beispiele für Verbindungen der Formel (1) seien die folgenden bevorzugten Verbindungen genannt:
Verbindung der Formel I a: 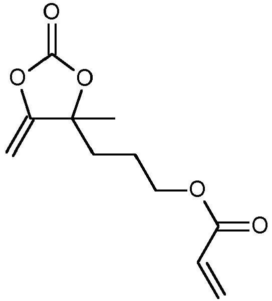
Verbindung der Formel I b: 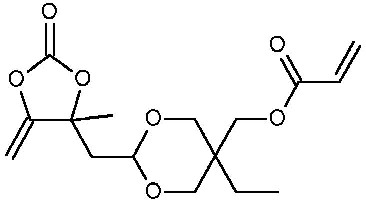
Verbindung der Formel I c: 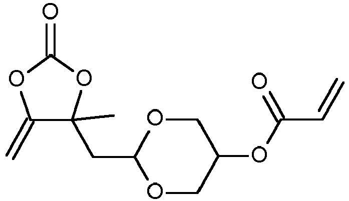
und Verbindung der Formel I d

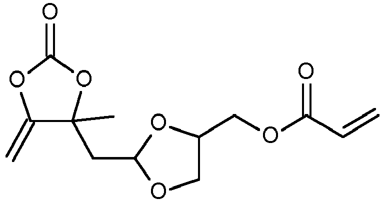

Herstellung von Verbindungen der Formel (I) mit R¹ = Formel (II)
Verbindungen der Formel (I) mit R¹ = Formel (II) können insbesondere durch ein Verfahren hergestellt werden, bei dem
- in einer ersten Stufe eine Verbindung mit einer endständigen Dreifachbindung mit einem Hydroxyalkanon oder Hydroxyalkanal umgesetzt wird, wobei sich die Dreifachbindung an die Carbonylgruppe des Hydroxyalkanon oder Hydroxyalkanal unter Ausbildung einer Dihydroxyverbindung addiert,
- in einer zweiten Stufe die Hydroxygruppe der erhaltenen Dihydroxyverbindung, welche nicht aus der Carbonylgruppe hervorgegangen ist, mit einer Schutzgruppe geschützt wird,
- in einer dritten Stufe mit Kohlendioxid der Ringschluss zur Carbonatgruppe erfolgt und
- in einer vierten Stufe die Schutzgruppe durch eine (Meth)acrylgruppe ersetzt wird.

### Zu Stufe 1

Die Umsetzung der ersten Stufe ist eine an sich bekannte Addition von Dreifachbindungen an eine Carbonylgruppe.

Geeignete Verbindungen mit einer endständigen Dreifachbindung sind insbesondere Verbindungen der Formel V

Y-CH≡CH,

wobei Y für ein H-Atom, eine Kohlenwasserstoffgruppe mit 1 bis 10 C- Atomen, z.B.eine Alkyl- oder Arylgruppe oder eine Schutzgruppe mit maximal 10 C-Atomen steht. Soweit es sich bei Y nicht um eine Schutzgruppe handelt, bestimmen die Substituenten des Y- substituierten C-Atoms die späteren Reste R3 und R4 in Formel I. Ausgehend von Formel V ist daher einer der Reste R3 oder R4 in Formel I ein H-Atom und der andere Y. Die bevorzugten Bedeutungen von Y entsprechen daher den obigen bevorzugten Bedeutungen von R3 bzw. R4.

Y kann jedoch auch für eine Schutzgruppe stehen. Schutzgruppen werden während oder nach der erfolgten Synthese wieder abgespalten, so dass in diesem Fall die späteren Reste R3 und R4 in Formel I beide für ein H-Atom stehen. Eine geeignete Schutzgruppe ist z.B.die Trimethylsilylgruppe (kurz TMS)

Bevorzugte Hydroxyalkanone oder Hydroxyalkanale sind Verbindungen der Formel VI 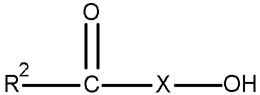 worin R2 für ein H-Atom oder eine C1- bis C10 Alkylgruppe steht.

R2 entspricht dem R2 in Formel I; in einer bevorzugten Ausführungsform ist R2 eine Methylgruppe.

X entspricht dem X in Formel II.

Zu den bevorzugten Bedeutungen von R2 und X wurden bereits oben Ausführungen gemacht.

Zur Durchführung der Additionsreaktion sind verschiedene Methoden bekannt. Vorzugsweise werden die Ausgangsverbindungen in Gegenwart einer starken Base umgesetzt. Bevorzugte starke Basen sind Metallalkoholate. Es handelt sich dabei vorzugsweise um Metallsalze aliphatischer Alkohole, insbesondere Metallsalze von C1- bis C8-, vorzugsweise C2- bis C6- Alkoholen, wie Ethanol, n-Propanol, iso-Propanol, n-Butanol oder tertiär-Butanol. Bei den Metallkationen der Metallalkoholate handelt es sich vorzugsweise um Alkali-kationen, z.B. die Kationen von Natrium oder Kalium. Als bevorzugtes Metallalkoholat seien z.B.Kalium-tertiärbutylat, Natriumtertiärbutylat, Kaliumisopropylat und Natriumisopropylat genannt.

Die Umsetzung wird vorzugsweise in Gegenwart eines Lösemittels durchgeführt. Als Lösemittel bevorzugt sind inerte Lösemittel; diese enthalten keine reaktiven Gruppen, welche mit den Ausgangsverbindungen reagieren. Besonders bevorzugt sind inerte, polare, aprotische Lösemittel. Als solche genannt seien z.B.cyclische Etherverbindungen, insbesondere THF.
Die Umsetzung ist im Allgemeinen exotherm, daher wird bei der Umsetzung vorzugsweise gekühlt. Die Temperatur des Reaktionsgemisches beträgt vorzugsweise maximal 50 °C, insbesondere maximal 25°C; vorzugsweise liegt sie zwischen 0 und 25°C.

Zur Aufarbeitung des erhaltenen Produktgemisches kann Wasser, gegebenenfalls Säure und gegebenenfalls ein unpolares organisches Lösemittel zugegeben. Soweit das Wertprodukt der 1.Stufe bereits eine eigenständige organische Phase ausbildet, kann auf das organische Lösemittel verzichtet werden. Es bilden sich zwei Phasen aus, von denen die organische Phase das Produkt der 1. Stufe (Additionsprodukte) enthält. Die organische Phase kann zur Entfernung von Wasser getrocknet werden. Lösemittel kann leicht destillativ abgetrennt werden. Das Produkt kann durch Vakuum-destillation in Reinform erhalten werden.
Alternativ kann die Aufarbeitung auch nach üblichen Verfahren der Kristallisation oder Extraktion erfolgen, insbesondere wenn das Produkt der 1.Stufe einen sehr hohen Siedepunkt hat.

### Zu Stufe 2

In der zweiten Stufe wird eine Hydroxygruppe der erhaltenen Dihydroxyverbindung mit einer Schutzgruppe geschützt. Die Hydroxygruppe, welche aus der Carbonylgruppe hervorgegangen ist, wird in der dritten Stufe mit CO₂ zum Carbonatring umgesetzt. Die schon vor der 1. Stufe vorhandene Hydroxygruppe der Ausgangsverbindung (kurz Ausgangs-Hydroxygruppe) wird geschützt, um Nebenreaktionen zu vermeiden.

Als Schutzgruppen geeignet sind insbesondere Estergruppen. Dazu wird die Ausgangs-Hydroxygruppe mit einer Säure oder einem Säurederivat umgesetzt. Bevorzugt ist die Umsetzung mit einer Carbonsäure, z.B.Ameisensäure oder Essigsäure, oder insbesondere mit einem Carbonsäurederivat, z.B. einem Carbonsäureanhydrid, Carbonsäurester oder Carbonsäurechlorid.

Die Ausgangs-Hydroxygruppe ist wesentlich reaktiver als die Hydroxygruppe, welche aus der Carbonylgruppe hervorgegangen ist. Daher bildet sich bei der Veresterung zunächst nur der Ester der Ausgangs-hydroxygruppe. Die Veresterung wird abgebrochen, bevor eine merkliche Esterbildung mit der anderen Hydroxygruppe beobachtet wird. Die Umsetzung kann durch Gaschromatographie kontrolliert werden.

Die Veresterung kann nach üblichen Verfahren durchgeführt werden. bei der Umsetzung wird die Temperatur vorzugsweise nur langsam gesteigert, so dass das Fortschreiten der Esterbildung gut chromatographisch verfolgt werden und die Umsetzung rechtzeitig abgebrochen werden kann.
Die Umsetzung kann z.B.bei Temperaturen von 0 bis 100°C, vorzugsweise liegt sie zwischen 0 und 40°C.

Der erhaltene Ester kann durch Destillation gereinigt werden. Bei hohen Siedepunkten kommt auch eine Aufarbeitung und Reinigung durch Kristallisation oder Extraktion in Betracht.

### Zu Stufe 3

In einer dritten Stufe erfolgt der Ringschluss mit Kohlendioxid zur Ausbildung der cyclischen Carbonatgruppe.

Dazu wird Kohlendioxid vorzugsweise gasförmig oder in überkritischem Zustand unter Druck mit dem Ester in Kontakt gebracht. Die Umsetzung wird daher vorzugsweise in einem Autoklaven durchgeführt.

Kohlendioxid kann auch in Gemisch mit Inertgas verwendet werden.

Die Umsetzung erfolgt vorzugsweise in Gegenwart von Katalysatoren. Vorzugsweise erfolgt sie in Gegenwart einer Base als Katalysator oder besonders bevorzugt in Gegenwart eines Katalysatorsystems aus einer Base und einem Metallsalz. Als Base sind Verbindungen mit mindestens einer tertiären Aminogruppe, z.B.mit ein bis drei tertiären Aminogruppen bevorzugt. Derartige Basen sind bekannt. Sie haben üblicherweise ein Molgewicht unter 500 g/mol, insbesondere unter 300 g/mol. Insbesondere handelt es sich dabei um aliphatische oder cycloaliphatische Verbindungen.

Als Basen genannt seien z.B.
TMTACN (N,N',N"-trimethyl-1,4,7-triazacyclononan),
PMDETA (Pentamethyldiethylentriamin)
TMEDA (Tetramethylethylendiamin)
DBU (1,8-Diazabicyclo[5.4.0]undec-7-en) oder
DBN (1,5-Diazabicyclo[4.3.0]non-5-en).

Bei dem Metallsalz handelt es sich vorzugsweise um Salze mit ein bis drei wertigen Kationen, insbesondere Kationen des Cu, Ag oder Au. Das Anion der Metallsalze ist vorzugsweise ein Carboxylat, insbesondere ein C1- bis C6-Carboxylat. Als bevorzugte Metallsalze genannt seien Silberacetat oder Kupferacetat.

Als Katalysatoren kommen auch Phosphane in Betracht. Insbesondere handelt es sich dabei um Trialkyl- oder Triarylphosphane. Diese können allein oder ebenfalls in Kombination mit einem Metallsalz eingesetzt werden.

Die Umsetzung wird vorzugsweise bei einem Druck von 1 bis 100 bar, insbesondere 5 bis 70 bar durchgeführt. Die Temperatur des Reaktionsgemisches beträgt vorzugsweise 10 bis 100°C, insbesondere 10 bis 80°C. Die Umsetzung kann z.B.durch Gaschromatographie überwacht werden.

Nach Abkühlung und Druckentspannung kann das erhaltene Produkt (ExoVC-Ester) aufgearbeitet werden. Es kann ein organisches Lösemittel, vorzugsweise ein inertes, hydrophobes organisches Lösemittel wie Dichlormethan oder Toluol, und wässrige Säure, z.B.wässrige HCl, zugegeben werden, so dass sich zwei Phasen bilden. Die organische Phase enthält das gewünschte Produkt. Aus der organischen Phase kann Wasser durch Trocknung entfernt werden. Lösemittel kann destillativ entfernt werden. Das Produkt kann durch Destillation gereinigt werden. Eine schonende und daher bevorzugte Destillation ist z.B.die Destillation in einem Dünnschichtverdampfer. Insbesondere sind dazu Dünnschichtverdampfer mit Wischersystem geeignet.

Alternativ kommt bei hohen Siedepunkten des Produkts der dritten Stufe auch eine Aufarbeitung und Reinigung durch Kristallisation oder Extraktion in Betracht.

### Zu Stufe 4

In der 4. Stufe wird das in der dritten Stufe erhaltene, geschützte ExoVC, z.B.der ExoVC-ester, mit einer (Meth)acrylverbindung zum ExoVC-(meth)acrylat der Formel I umgesetzt.

Als (Meth)acrylverbindung wird insbesondere ein Alkyl(meth)acrylat, z.B.Methyl(meth)acrylat, verwendet. Die Schutzgruppe, hier das Formiat, wird durch die (Meth)acrylgruppe ersetzt, wobei das entsprechende Alkylformiat freigesetzt wird.

Diese Reaktion kann durch verschiedene Methoden durchgeführt werden, welche dem Fachmann bekannt sind. Vorzugsweise wird sie enzymatisch durchgeführt.

Als Enzyme eignen sich dazu Lipasen. Lipasen katalysieren den Fettabbau bzw. Fettbildung aus Fettsäuren und Glycerin und eignen sich daher auch als Katalysatoren für Veresterungen bzw. Umesterungen oder entsprechende Umesterungs-reaktionen, bei denen zwei Ester ihren aus der Veresterungsreaktion stammenden Rest des verwendeten Alkohols tauschen.

Eine geeignete Lipase ist z.B.das Enzym Candida Antartica Lipase B. Die Lipasen werden vorzugsweise in einer immobilisierten Form verwendet. Immobilisierte Lipasen sind solche, die an einen Träger gebunden sind. Geeignete Träger sind natürliche organische Polymere wie Cellulose, synthetische organische Polymere wie Polystyrol oder anorganische Substanzen wie Tone oder Silikate.

Die enzymatisch katalysierte Reaktion kann z.B. bei einer Temperatur des Reaktionsgemisches von 0 bis 100°C, vorzugsweise 20 bis 80°C, besonderes bevorzugt 30 bis 60°C erfolgen. Die Umsetzung wird einfacherweise bei Normaldruck durchgeführt, eine Druckerhöhung oder-absenkung ist nicht notwendig, aber auch nicht nachteilig.

Bei der Reaktion handelt es sich um eine Gleichgewichtsreaktion. Die bei der Reaktion frei gesetzte Verbindung wird daher vorzugsweise dem Gemisch entzogen, sei es durch Abdestillieren, z.B.des freigesetzten Alkylformiats, oder auch durch Absorption, z.B.an einem Molekularsieb.

Die Entfernung der frei gesetzten Verbindung kann kontinuierlich, z.B.durch ein dauerhaftes Vakuum erfolgen. Alternativ kann die Umsetzung nach einer bestimmten Zeit unterbrochen werden, um die frei gesetzte Verbindung zu entfernen, und danach wieder fortgesetzt werden.

Abschließend kann das Enzym durch Filtration aus der Reaktionsmischung abgetrennt werden und die Mischung durch destillative Abtrennung der nicht umsetzten Ausgangsstoffe oder entstandenen Nebenprodukte gereinigt werden.

Die Umsetzung über alle vier Stufen verläuft insgesamt problemlos und in einfacher Weise und eignet sich daher auch für einen industriellen Maßstab. Das ExoVC-(meth)acrylat wird mit hoher Ausbeute und Selektivität erhalten.

Eine schematische Darstellung der vier Stufen findet sich auch im Herstellungsbeispiel 2.1.

Herstellung von Verbindungen der Formel (I) mit R¹ = Formel (III)
Verbindungen der Formel (I) mit R¹ = Formel (III) können insbesondere durch Verfahren hergestellt werden, bei dem
- in einer ersten Stufe eine Verbindung mit einer endständigen Dreifachbindung mit einem Alkanon oder Alkanal, welches eine Acetalgruppe enthält, umgesetzt wird, wobei sich die Dreifachbindung an die Carbonylgruppe des Alkanon oder Alkanals unter Ausbildung einer Hydroxyverbindung addiert,
- in einer zweiten Stufe mit Kohlendioxid der Ringschluss zur Carbonatgruppe erfolgt,
- in einer dritten Stufe der Ringschluss zum 1,3-Dioxanring durch Umsetzung der Acetalgruppe mit einer Verbindung mit insgesamt mindestens drei Hydroxylgruppen wobei sich zwei der Hydroxylgruppen in 1,3 Stellung befinden, durchgeführt wird und
- in einer vierten Stufe die (Meth)acrylgruppe durch eine Veresterung oder Umesterung der verbleibenden Hydroxylgruppe eingeführt wird.

### Zu Stufe 1

Die Umsetzung der ersten Stufe ist eine an sich bekannte Addition von Dreifachbindungen an eine Carbonylgruppe.

Geeignete Verbindungen mit einer endständigen Dreifachbindung sind insbesondere Verbindungen der Formel V, wie sie oben aufgeführt sind. Die obigen Ausführungen zu Formel V und den bevorzugten Verbindungen der Formel gelten hier entsprechend.

Die verwendeten Alkanone oder Alkanale enthalten eine Acetalgruppe.

Bevorzugte Alkanone oder Alkanale mit einer Acetalgruppe sind solche der Formel VII 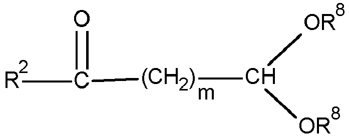 worin R² für ein H-Atom oder eine C1- bis C10 Alkylgruppe steht, m für 0 oder eine ganze Zahl von 1 bis 10 steht. m entspricht dem m in der obigen Formel III und hat die entsprechenden Bedeutungen und bevorzugten Bedeutungen. 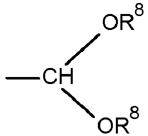 steht für die Acetalgruppe, wobei R⁸ eine Kohlenwasserstoffgruppe, insbesondere eine C₁- bis C₁₀-Alkylgruppe, besonders bevorzugt eine Methylgruppe, bedeutet.

R² entspricht dem R² in Formel I und hat die entsprechenden Bedeutungen und bevorzugten Bedeutungen; in einer bevorzugten Ausführungsform ist R² eine Methylgruppe.

Zur Durchführung der Additionsreaktion gelten analog die obigen Ausführungen zur Stufe 1 bei der Herstellung von Verbindungen der Formel I mit R¹ = Formel II.

### Zu Stufe 2

In Stufe 2 erfolgt der Ringschluss. Hier gelten analog die obigen Ausführungen zur Stufe 3 bei der Herstellung von Verbindungen der Formel I mit R¹ = Formel II.

Produkt der zweiten Stufe ist eine Verbindung mit einer cyclischen Carbonatgruppe und einer Acetalgruppe.

### Zu Stufe 3

In der 3. Stufe erfolgt der Ringschluss zum 1,3 Dioxanring. Dazu wird das Produkt der zweiten Stufe mit einer Verbindung mit insgesamt mindestens drei Hydroxylgruppen, vorzugsweise genau drei Hydroxylgruppen, wobei sich zwei der Hydroxylgruppen in 1,3 Stellung befinden, umgesetzt. Die beiden Hydroxylgruppen in 1,3 Stellung bilden durch Umsetzung mit der Acetalgruppe den 1,3 Dioxanring.

Diese Umsetzung kann nach üblichen Methoden durchgeführt werden.

Bei der Verbindung mit den mindestens drei Hydroxylgruppen handelt es sich vorzugsweise um eine Verbindung der Formel VIII 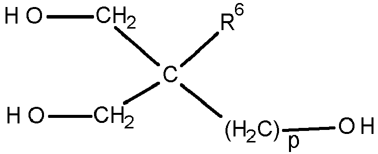 R⁶ und p entsprechen R⁶ und p in obiger Formel III und haben daher die entsprechenden Bedeutungen und bevorzugten Bedeutungen.

Insbesondere handelt es sich bei der Verbindung der Formel VIII um Trimethylolpropan.

Die Umsetzung kann in Gegenwart oder Abwesenheit von Lösemittel durchgeführt werden. Vorzugsweise erfolgt sie in Gegenwart von Lösemittel. Das Lösemittel wird dabei vorzugsweise so gewählt, dass alle Ausgangsstoffe möglichst vollständig gelöst werden. Bei der Umsetzung wird der Alkohol HO-R⁸ abgespalten, insbesondere handelt es sich dabei um Methanol. Bei Verwendung eines hochsiedenden Lösemittels wie Toluol kann Methanol leicht destillativ entfernt werden. Der Alkohol HO-R⁸, insbesondere Methanol, kann jedoch auch in der Reaktionsmischung verbleiben; daher sind auch Lösemittel mit tieferem Siedepunkt oder polare Lösemittel, wie Acetonitril geeignet.

Vorzugsweise erfolgt die Umsetzung in Gegenwart von Katalysatoren. Als Katalysatoren geeignet sind z.B.organische oder anorganische Säuren. Genannt seien exemplarisch Methansulfonsäure und p-Toluolsulfonsäure.

Das bei der Umsetzung verwendete Lösemittel kann gegebenenfalls vor der weiteren Aufarbeitung entfernt werden. Die Aufarbeitung kann wiederum durch eine Phasentrennung erfolgen. Dazu wird ein geeignetes Lösemittel, welches das Wertprodukt der 3. Stufe möglichst vollständig löst und mit Wasser nicht mischbar ist zugegeben. Als Lösemittel kommen z.B.Toluol oder MTBE (Methyltertiärbutylether) in Betracht. Die erhaltene organische Phase kann mit Wasser einmal oder mehrfach gewaschen werden. Die wässrige Phase wird jeweils verworfen. Die organische Phase kann durch Destillation oder Kristallisation gereinigt werden.

### Zur 4. Stufe

In der 4. Stufe wird schließlich die Verbindung der Formel I mit R¹= Formel III durch eine Umesterung erhalten. Diese 4. Stufe entspricht der 4. Stufe bei der Herstellung von Verbindungen der Formel I mit R¹ = Formel II, nur mit dem Unterschied, dass hier nicht von einem geschützten Alkohol ausgegangen wird, sondern von einem ungeschützten. Es gelten daher alle diesbezüglichen obigen Ausführungen entsprechend.

Eine schematische Darstellung der vier Stufen findet sich auch im Herstellungsbeispiel 2.2.

In einer alternativen Verfahrensweise könnte man die Reihenfolge der Stufen ändern und z.B. die Reihenfolge der Stufen 2 und 3 umkehren, so dass sich die Reihenfolge 1-3-2-4 der vorstehenden Stufen ergibt. Dann würde zunächst die Umacetalisierung der Verbindung der Formel VIII mit der Verbindung der Formel VIII durchgeführt. Vorzugsweise wird dabei die dritte Hydroxylgruppe der Verbindung VIII, welche an der Umacetalisierung nicht teilnehmen soll, durch eine Schutzgruppe geschützt. Erst danach erfolgt dann der Ringschluss mit CO₂.

Bevorzugt ist jedoch die obige, numerische Reihenfolge der Stufen von Stufe 1, über Stufe 2, dann 3 zu Stufe 4.

Herstellung von Verbindungen der Formel (I) mit R¹ = Formel (IV)
Verbindungen der Formel (I) mit R¹ = Formel (IV) können insbesondere durch Verfahren hergestellt werden, bei dem
- in einer ersten Stufe eine Verbindung mit einer endständigen Dreifachbindung mit einem Alkanon oder Alkanal, welches eine Acetalgruppe enthält, umgesetzt wird, wobei sich die Dreifachbindung an die Carbonylgruppe des Alkanon oder Alkanals unter Ausbildung einer Hydroxyverbindung addiert,
- in einer zweiten Stufe mit Kohlendioxid der Ringschluss zur Carbonatgruppe erfolgt,
- in einer dritten Stufe der Ringschluss zum 1,3-Dioxolanring durch Umsetzung der Acetalgruppe mit einer Verbindung mit insgesamt mindestens drei Hydroxylgruppen wobei sich zwei der Hydroxylgruppen in 1,2 Stellung befinden, durchgeführt wird und
- in einer vierten Stufe die (Meth)acrylgruppe durch eine Veresterung oder Umesterung der verbleibenden Hydroxylgruppe eingeführt wird.

Diese vier Stufen entsprechen den vier Stufen bei der vorstehend beschriebenen Herstellung von Verbindungen der Formel I mit R¹= Formel III. daher gelten die vorstehenden Ausführungen zur Herstellung von Verbindungen der Formel I mit R¹= Formel III entsprechend, soweit im Folgenden nichts anderes festgestellt wird.

Der wesentliche Unterschied besteht naturgemäß in der Wahl der Verbindung mit den mindestens drei Hydroxygruppen in der 3. Stufe, da ein 1,3 Dioxolanring (Fünfring) statt eines 1,3 Dioxanrings (Sechsring) hergestellt wird.

Entsprechend stehen zwei der Hydroxylgruppen der Verbindung in 1,2 Stellung.

Eine bevorzugte Verbindung mit drei derartige Hydroxylgruppen ist Glycerin.

### Verwendung in Epoxidharz-zusammensetzungen

Verbindungen der Formel (I) eignen sich als Zusatzstoffe zu Epoxidharz-zusammensetzungen. Insbesondere wirken sie dabei als Reaktivverdünner. Geeignete Epoxidharz-zusammensetzungen sind in WO 2013/050311 und WO 2011/157671 beschrieben. Die Verbindungen der Formel (I) können die in WO 2013/050311 und WO 2011/157671 beschriebenen cyclischen Carbonate in den Epoxidharz-zusammensetzungen ersetzen.

Epoxidharz-zusammensetzungen enthalten im Allgemeinen
a) Verbindungen der Formel (I) und
b) Verbindungen mit mindestens einer Epoxygruppe (kurz Epoxyverbindungen) und
c) gegebenenfalls Härter für Epoxyverbindungen und
d) gegebenenfalls weitere Bestandteile
zu den Verbindungen der Formel (I)

Zu den Verbindungen der Formel (I) gilt das oben gesagte. Die oben als bevorzugt bezeichneten Verbindungen sind auch in den Epoxidharz-zusammensetzungen bevorzugt.

Verbindungen der Formel I können in den Epoxidharz-zusammensetzungen in beliebigen Mengen enthalten sein. Sie können in geringen Mengen, aber auch in deutlichem Überschuss enthalten sein. Die Möglichkeit, Verbindungen der Formel I im Unterschuss, aber auch im Überschüss zu verwenden, ist ein erheblicher Vorteil.

Die Verbindung(en) der Formel I können z.B. in einer Gesamtmenge von mindestens 0,1 Gewichtsteilen, insbesondere mindestens 0,5 Gewichtsteilen, besonders bevorzugt mindestens 1 Gewichtsteilen, bzw. von mindestens 2 oder mindestens 5 Gewichtsteilen bezogen auf 100 Gewichtsteil der Epoxyverbindungen b), verwendet werden. Die Verbindung(en) der Formel I können in einer Gesamtmenge z.B. von maximal 10000 Gewichtsteilen, vorzugsweise maximal 1000 Gewichtsteilen, insbesondere maximal 200 Gewichtsteilen bzw. 100 Gewichtsteilen bezogen auf 100 Gewichtsteile der Epoxyverbindungen b), verwendet werden.

Die Epoxidharz-zusammensetzungen enthalten daher Verbindungen der Formel I insbesondere in Mengen von z.B.0,1 bis 10000 Gewichtsteilen, bevorzugt von 0,1 bis 1000 Gewichtsteilen, besonders bevorzugt von 0,5 bis 200 Gewichtsteilen auf 100 Gewichtsteile Epoxyverbindungen b).

### Zu den Epoxyverbindungen

Als Epoxyverbindungen in Betracht kommen insbesondere solche, die üblicherweise in härtbaren Epoxidharz-zusammensetzungen eingesetzt werden. Genannt seien insbesondere Verbindungen mit 1 bis 10 Epoxidgruppen, vorzugsweise mit mindestens zwei Epoxidgruppen im Molekül. Der Gehalt an Epoxid-Gruppen in typischen Epoxidharzen liegt im Bereich von 120 bis 3000 g/Äquivalent, gerechnet als sogenanntes Epoxidäquivalent gemäß DIN 16945.

Hierunter bevorzugt sind sogenannte Glycidyl-basierte Epoxyverbindungen, insbesondere solche, die durch Veretherung aromatischer, aliphatischer oder cycloaliphatischen Polyole mit Epichlorhydrin hergestellt werden. Derartige Glycidyl-basierte Epoxyverbindungen werden auch als Polyglycidylether von aromatischen, aliphatischen oder von cycloaliphatischen Polyolen bezeichnet.

Bei den Epoxyverbindungen kann es sich um bei 20°C, 1 bar flüssige Verbindungen (kurz Flüssigharze), oder um bei 20°C, 1 bar feste Verbindungen (kurz Festharze) oder um Mischungen davon handeln. Flüssigharze unterscheiden sich von Festharzen durch geringere Viskosität. Zudem weisen Flüssigharze in der Regel einen höheren Anteil an Epoxidgruppen und dementsprechend ein niedrigeres Epoxidäquivalent auf.

Der Gehalt an Epoxidgruppen in typischen Flüssigharzen liegt üblicherweise im Bereich von 120 bis 200 g/Äquivalent und der der Festharze im Bereich von 450-3000 g/Äquivalent, gerechnet als sogenanntes Epoxidäquivalent gemäß DIN 16945.

Die Viskosität der Flüssigharze liegt bei 25°C üblicherweise im Bereich von 1 bis 20 Pas, bevorzugt im Bereich von 5 bis 15 Pas. Die Viskosität der der Festharze liegt bei 25°C üblicherweise im Bereich 5 bis 40 Pas, bevorzugt im Bereich von 20 bis 40 Pas. Die hier angegebenen Viskositäten sind die gemäß DIN 53015 bei 25°C als 40%ige Lösungen der Harze in Methylethylketon bestimmten Werte.

Geeignete Epoxyverbindungen sind beispielsweise die unter den Markenbezeichnungen EPILOX®, EPONEX®, EPIKOTE®, EPONOL®, D.E.R, ARALDIT® oder ARACAST® im Handel erhältlich.

In einer bevorzugten Ausführungsform ist das Epoxidharz ausgewählt aus Polyglycidethern aromatischer Polyole.

Beispiele hierfür sind die vom Diglycidylether des Bisphenols A abgeleitetenen Verbindungen (R' = CH3) und die vom Bisphenol F abgeleiteten Harze (R' = H), welche sich durch die folgende allgemeine Formel beschreiben lassen: 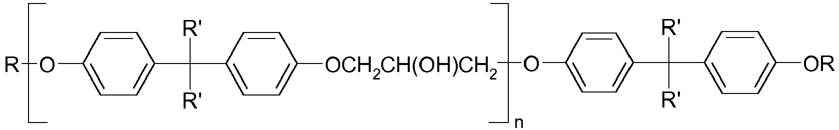 R = 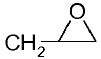 R' = H oder CH₃
In der Formel gibt der Parameter n die Anzahl der Wiederholungseinheiten an, wobei der Mittelwert von n mit dem jeweiligen mittleren Molekulargewicht korrespondiert.

n kann z.B.einen Wert von 0 bis 10 haben.

Beispiele für Epoxyverbindungen auf Basis von Polyglycidylethern aromatischer Polyole sind weiterhin Glycidylether von Phenol- und Kresol-basierten Novolaken. Novolake werden durch die säurekatalysierte Kondensation von Formaldehyd und Phenol oder Kresol hergestellt. Durch Umsetzung der Novolake mit Epichlorhydrin erhält man die Glycidylether der Novolake. Insbeondere kommen auch Gemische von unterschiedlichen Polyglycidylethern aromatischer Polyole in Betracht.

In einer anderen bevorzugten Ausführungsform der Erfindung ist das Epoxyverbindungen ausgewählt aus Polyglycidylethern cycloaliphatischer Polyole und den Polyglycidylestern cycloaliphatischer Polycarbonsäuren. Beispiele für Polyglycidylether von cycloaliphatischen Polyolen sind die Kernhydrierungsprodukte von Polyglycidylethern auf Basis von Bisphenol-A, die Kernhydrierungsprodukte von Polyglycidylethern auf Basis von Bisphenol-F, die Kernhydrierungsprodukte von Polyglycidylethern auf Basis von Novolaken und deren Gemische. Derartige Verbindungen werden üblicherweise durch selektive Hydrierung der aromatischen Ringe in den zuvor genannten aromatischen Polyglycidylethern hergestellt. Beispiele für solche Produkte sind das P 22-00 der Fa. LeunaHarze und Eponex 1510 der Fa. Hexion. Beispiele für Polyglycidylester von cycloaliphatischen Polycarbonsäuren ist Hexahydrophthalsäurediglycidylester Als Epoxyverbindungen für Lackformulierungen sind auch epoxidgruppenhaltige Polyacrylatharze geeignet. Diese werden in der Regel durch Copolymerisation von mindestens einem ethylenisch ungesättigten Monomer, das mindestens eine Epoxidgruppe, insbesondere in Form einer Glycidylethergruppe, im Molekül enthält, mit mindestens einem weiteren ethylenisch ungesättigten Monomer, das keine Epoxidgruppe im Molekül enthält, hergestellt, wobei vorzugsweise wenigstens eines der Comonomere ein Ester der Acrylsäure oder Methacrylsäure ist. Beispiele für die ethylenisch ungesättigte Monomere, die mindestens eine Epoxidgruppe im Molekül enthalten, sind Glycidylacrylat, Glycidylmethacrylat und Allylglycidylether. Beispiele für ethylenisch ungesättigte Monomere, die keine Epoxidgruppe im Molekül enthalten, sind Alkylester der Acryl und Methacrylsäure, die 1 bis 20 Kohlenstoffatome im Alkylrest enthalten, insbesondere Methylacrylat, Methylmethacrylat, Ethylacrylat, Ethylmethacrylat, Butylacrylat, Butylmethacrylat, 2-Ethylhexylacrylat und 2-Ethylhexylmethacrylat. Weitere Beispiele für ethylenisch ungesättigte Monomere, die keine Epoxidgruppen im Molekül enthalten, sind Säuren, wie z.B.Acrylsäure und Methacrylsäure. Säureamide, wie z.B.Acrylsäure- und Methacrylsäureamid, vinylaromatische Verbindungen, wie Styrol, Methylstyrol und Vinyltoluol, Nitrile, wie Acrylnitril und Methacrylnitril, Vinyl- und Vinylidenhalogenide, wie Vinylchlorid und Vinylidenfluorid, Vinylester, wie z.B.Vinylacetat und hydroxylgruppenhaltige Monomere, wie z.B.Hydroxyethylacrylat und Hydroxyethylmethacrylat. Das epoxidgruppenhaltige Polyacrylatharz weist üblicherweise ein Epoxidäquivalentgewicht von 400 bis 2500, vorzugsweise 500 bis 1500, besonders bevorzugt 600 bis 1200 auf. Das zahlenmittlere Molekulargewicht (gelpermeationschromatographisch unter Verwendung eines Polystyrolstandards bestimmt) liegt typischerweise im Bereich von 1000 bis 15 000, vorzugsweise von 1200 bis 7000, besonders bevorzugt von 1500 bis 5000. Die Glasübergangstemperatur (TG) liegt typischerweise im Bereich von 30 bis 80°C, vorzugsweise von 40 bis 70°C, besonders bevorzugt von 50 bis 70°C (gemessen mit Hilfe der Differentialkalorimetrie (DSC)). Epoxidgruppenhaltige Polyacrylatharze sind bekannt (vgl. z.B.EP-A-299 420, DE-B-22 14 650, DE-B-27 49 576, US-A-4,091,048 und US-A-3,781,379). Beispiele für solche Harze sind Epon 8021, Epon 8111, Epon 8161 der Fa. Hexion.

Die Epoxyverbindungen können sich auch von anderen Epoxiden ableiten (Nichtglycidylether-Epoxidharze). Hierzu zählen insbesondere Verbindungen, einschließlich Oligomeren und Polymeren, die wenigstens eine, insbesondere mehrere epoxidierte cycloaliphatische Gruppen, insbesondere 7-Oxabicyclo-[4.1.0]-heptyl-Gruppen aufweisen, die durch Epoxidierung von Verbindungen mit Cyclohexenylgruppen erhältlich sind. Beispiele für die Epoxidierungsprodukte von Verbindungen mit wenigstens einer cycloolefinischen Gruppe sind 4-Epoxyethyl-1,2-epoxycyclohexan und die Verbindung der folgenden Formel: 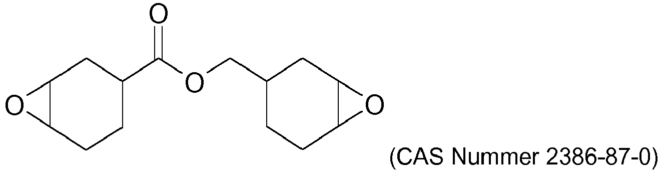 die beispielsweise von der Fa. Cytec unter der Bezeichnung Uvacure 1500 vertrieben wird. Bevorzugt werden die Verbindungen eingesetzt, die wenigstens eine, insbesondere mehrere epoxidierte cycloaliphatische Gruppen, insbesondere 7-Oxabicyclo-[4.1.0]-heptyl-Gruppen aufweisen, die durch Epoxidierung von Verbindungen mit Cyclohexenylgruppen erhältlich sind, und deren Oligomere nicht alleine sondern in Kombination mit einer oder mehrerer der vorgenannten Substanzen, die wenigstens zwei Glycidylethergruppen im Molekül aufweisen.

Die vorstehenden Epoxidharze können jeweils als alleinige Epoxidharze oder als Gemisch eingesetzt werden.

### Zu den Härtern für Epoxyverbindungen

Die Epoxidharz-zusammensetzung, welche Epoxidharze, eine oder mehrere Verbindungen der Formel I und gegebenenfalls weitere Bestandteile enthält, wird durch Zugabe von Härtern, welche mit den Epoxidgruppen reagieren, gehärtet.

Bei den Härtern handelt es sich vorzugsweise um Verbindungen mit mindestens einer primären oder sekundären Aminogruppe (kurz Amino-Härter).

Amino-Härter vernetzen Epoxidharze durch Reaktion der primären oder sekundären Aminogruppen mit den Epoxidgruppen der Epoxyverbindungen. Derartige Amino-härter haben vorzugsweise mindestens zwei Aminogruppen, im Allgemeinen haben sie 2 bis 6, insbesondere 2 bis 4 Aminogruppen. Die Amino-Härter können ausschließlich primäre Aminogruppem, ausschließlich sekundäre Aminogruppen oder sowohl primäre als auch sekundäre Aminogruppen enthalten.

Übliche Amino-Härter sind beispielsweise
- aliphatische Polyamine wie Ethylendiamin, 1,2- und 1,3-Propandiamin, Neopentandiamin, Hexamethylendiamin, Octamethylendiamin, 1,10-Diaminodecan, 1,12-Diaminododecan, Diethylentriamin, Triethylentetramin, Tetraethylenpentamin, Trimethylhexamethylendiamin, 1-(3-Aminopropyl)-3-aminopropan, 1,3-Bis-(3-aminopropyl)propan, 4-Ethyl-4-methyl-amino-1-octylamin und dergleichen;
- cycloaliphatische Diamine, wie 1,2-Diaminocyclohexan, 1,3-Bis(aminomethyl)cyclohexan, 1-Methyl-2,4-diaminocyclohexan, 4-(2-Aminopropan-2-yl)-1-methylcyclohexan-1-amin, Isophorondiamin, 4,4'-Diaminodicyclohexylmethan, 3,3'-Dimethyl-4,4'-diaminodicyclohexylmethan, 4,8-Diamino-tricyclo[5.2.1.0]decan, Norbornandiamin, Menthandiamin, Menthendiamin und dergleichen;
- aromatische Diamine, wie Toluylendiamin, Xylylendiamin, insbesondere meta-Xylylendiamin, Bis(4-aminophenyl)methan (MDA oder Methylendianilin), Bis(4-aminophenyl)sulfon (auch als DADS, DDS oder Dapson bekannt) und dergleichen;
- cyclische Polyamine, wie Piperazin, N-Aminoethylpiperazin und dergleichen;
- Polyetheramine, insbesondere difunktionelle und trifunktionelle primäre Polyetheramin auf der Basis von Polypropylenglykol, Polyethylenglykol, Polybutylenoxid, Poly-(1,4-butandiol), Poly-THF oder Polypentylenoxid, z.B.4,7,10-Trioxatridecan-1,3-diamin, 4,7,10-Trioxatridecan-1,13-diamin, 1,8-Diamino-3,6-dioxaoctan (XTJ-504 von Huntsman), 1,10-Diamino-4,7-dioxadecan (XTJ-590 von Huntsman), 1,12-Diamino-4,9-dioxadodecan (BASF SE), 1,3-Diamino-4,7,10-trioxatridecan (BASF), primäre Polyetheramine auf der Basis von Polypropylenglykol mit einer mittleren Molmasse von 230 wie z.B.Polyetheramine D 230 (BASF SE) oder Jeffamine® D 230 (Huntsman), difunktionelle, primäre Polyetheramine auf der Basis von Polypropylenglykol mit einer mittleren Molmasse von 400, z.B.Polyetheramine D 400 (BASF SE) oder Jeffamine® XTJ 582 (Huntsman), difunktionelle, primäre Polyetheramine auf Basis von Polypropylenglykol mit einer mittleren Molmasse von 2000 wie z.B.Polyetheramine D 2000 (BASF SE), Jeffamine® D2000 oder Jeffamine® XTJ 578 (Huntsman), difunktionelle, primäre Polyetheramine auf der Basis von Propylenoxid mit einer mittleren Molmasse von 4000 wie z.B.Polyetheramin D 4000 (BASF SE), trifunktionelle, primäre Polyetheramine hergestellt durch Reaktion von Propylenoxid mit Trimethylolpropan gefolgt durch eine Aminierung der endständigen OH-Gruppen mit einer mittleren Molmasse von 403 wie z.B.Polyetheramine T 403 (BASF SE) oder Jeffamine® T 403 (Huntsman), trifunktionelle, primären Polyetheramin hergestellt durch Reaktion von Propylenoxid mit Glycerin gefolgt durch eine Aminierung der endständigen OH-Gruppen mit einer mittleren Molmasse von 5000 wie z.B.Polyetheramine T 5000 (BASF SE) oder Jeffamine® T 5000 (Huntsman), aliphatische Polyetheramine, die aus einem mit Propylenoxid gepfropftem Polyethylenglykol aufgebaut sind und eine mittlere Molmasse von 600 aufweisen, wie z.B.Jeffamine® ED-600 bzw. Jeffamine® XTJ 501 (jeweils Huntsman), aliphatische Polyetheramine, die aus einem mit Propylenoxid gepfropftem Polyethylenglykol aufgebaut sind und eine mittlere Molmasse von 900 aufweisen, wie z.B.Jeffamine® ED-900 (Huntsman), aliphatische Polyetheramine, die aus einem mit Propylenoxid gepfropftem Polyethylenglykol aufgebaut sind und eine mittlere Molmasse von 2000 aufweisen, wie z.B.Jeffamine® ED-2003 (Huntsman), difunktionelle, primäre Polyetheramin, hergestellt durch Aminierung eines mit Propylenoxid gepfropften Diethylenglykols mit einer mittleren Molmasse von 220, wie z.B.Jeffamine® HK-511 (Huntsman), aliphatische Polyetheramine auf der Basis eines Copolymers aus Poly(tetramethylenetherglycol) und Polypropylenglycol mit einer mittleren Molmasse von 1000 wie z.B.Jeffamine® XTJ-542 (Huntsman), aliphatische Polyetheramine auf der Basis eines Copolymers aus Poly(tetramethylenetherglycol) und Polypropylenglycol mit einer mittleren Molmasse von 1900 wie z.B.Jeffamine® XTJ-548 (Huntsman), aliphatische Polyetheramine auf der Basis eines Copolymers aus Poly(tetramethylenetherglycol) und Polypropylenglycol mit einer mittleren Molmasse von 1400 wie z.B.Jeffamine® XTJ-559 (Huntsman), Polyethertriamine auf der Basis eines mit Butylenoxid gepfropftem mindestens dreiwertigen Alkohols mit einer mittleren Molmasse von 400, wie z.B.Jeffamine® XTJ-566 (Huntsman), aliphatische Polyetheramine hergestellt durch Aminierung von mit Butylenoxid aufgepfropften Alkoholen mit einer mittleren Molmasse von 219, wie z.B.Jeffamine® XTJ-568 (Huntsman), Polyetheramine auf der Basis von Pentaerythrit und Propylenoxid mit einer mittleren Molmasse von 600 wie z.B.Jeffamine® XTJ- 616 (Huntsman), Polyetheramine auf der Basis von Triethylenglykol mit einer mittleren Molmasse von 148, z.B.Jeffamine® EDR-148 (Huntsman), difunktionelle, primäre Polyetheramine hergestellt durch Aminierung eines mit Propylenoxid gepfropften Ethylenglykols, mit einer mittleren Molmasse von176 wie z.B.Jeffamine® EDR-176 (Huntsman), sowie Polyetheramine hergestellt durch Aminierung von PolyTHF mit einer mittleren Mollmasse von 250, z.B.PolyTHF-Amin 350 (BASF SE) und Mischungen dieser Amine.
- Polyamidoamine (Amidopolyamine), die durch die Reaktion von Polycarbonsäuren, insbesondere Dicarbonsäuren wie Adipinsäure oder dimeren Fettsäuren (z.B.dimere Linolsäure) mit niedermolekularen Polyaminen, wie Diethylentriamin, 1-(3-Aminopropyl)-3-aminopropan oder Triethylentetramin oder anderen Diaminen wie die zuvor genannten aliphatischen oder cycloaliphatischen Diamine, erhältlich sind oder alternativ durch Michael-Addition von Diaminen an Acrylsäureester und anschließende Polykondensation der erhaltenen Aminosäureester erhältlich sind,
- Phenalkamine (auch Phenolalkanamine), worunter Phenol oder Phenolderivate verstanden werden, welche an mindestens einem C-Atom des Ringsystems durch Kohlenwasserstoffgruppen substituiert sind, welche primäre oder sekundäre Aminogruppen enthalten; abgesehen von der Hydroxylgruppe des Phenols oder Phenolderivats und den primären oder sekundären Aminogruppen enthalten die Phenalkamine keine weiteren funktionellen Gruppen. Insbesondere enthalten die Phenalkamine sowohl primäre als auch sekundäre Aminogruppen. Gut geeignete Phenalkamine enthalten vorzugsweise insgesamt 2 bis 10, insbesondere 2 bis 8 und in einer besondere Ausführungsform 4 bis 6 derartiger Aminogruppen; bevorzugt handelt sind es sich um Phenalkamine auf Basis von Cardanol, welches im Cashew-Schalenöl enthalten ist; auf Cardanol basierende Phenalkamine sind an mindestens einem, vorzugsweise an ein bis drei C-Atomen des Ringsystems durch vorstehend beschriebene, primäre oder sekundäre Aminogruppen enthaltende, vorzugsweise aliphatische Kohlenwasserstoffgruppen substituiert. Insbesondere finden sich diese Substituenten in ortho- oder para-Stellung zur Hydroxylgruppe; Phenalkamine können durch Mannich-Reaktion aus dem Phenol oder Phenolderivat, einem Aldehyd, und einer Verbindung mit mindestens einer primären oder sekundären Aminogruppe hergestellt werden. Bei den Phenalkaminen handelt es sich daher um Mannich-Basen oder Addukte von Aminoverbindungen, insbesondere einer der vorstehenden Amino-verbindungen an Epoxidverbindungen und
- Addukte von Aminoverbindungen an Epoxidverbindungen; derartige Addukte sind Umsetzungprodukte von Epoxidverbindungen mit einem Überschuss an Aminoverbindungen, so dass alle Epoxidgruppen umgesetzt sind und die erhaltenen Verbindungen noch primäre oder sekundäre Aminogruppen aufweisen und diese Addukte daher entsprechend als Aminohärter verwendet werden können. Als Epoxidverbindungen sind hier solche mit einer oder zwei Epoxidgruppen bevorzugt. Als Aminoverbindungen werden für Herstellung von Addukte vorzugsweise niedermolekulare Aminoverbindungen mit primären Aminogruppen eingesetzt, insbesondere solche wie sie weiter unter auch als Aminverbindungen H1 (Cohärter) beschrieben sind. Als Beispiele für Addukte seien die Addukte von Xylenendiamin (MXDA), Isophorondiamin (IPDA) oder Diethylentriamin an Bisphenol A oder Bisphenol F genannt.

Es können auch beliebige Mischungen aus verschiedenen Amino-Härtern verwendet werden.

In einer bevorzugten Ausführungsform können Mischungen von den nachstehend definierten Amino-Härtern H1 und H2 verwendet werden.

Diese Amino-Härter H1 werden im Nachfolgenden auch Co-Härter genannt, während andere Amino-Härter, die nicht unter die vorstehende Definition der Amino-Härter H1 fallen, im Nachfolgenden Amino-Härter H2 genannt werden.

### Zu den Amino-Härtern H1 (Co-Härter)

Amino-Härter H1 sind aliphatische, cycloaliphatische oder aromatische Aminverbindungen mit 1 bis 4 primären Aminogruppen und gegebenenfalls weiteren funktionellen Gruppen, ausgewählt aus sekundären Aminogruppen, tertiären Aminogruppen und Hydroxylgruppen, wobei die primären Aminogruppen im Falle der cycloaliphatischen und aromatischen Aminverbindungen als Aminomethylengruppen (H2N-CH2-) an das cycloaliphatische oder aromatische Ringsystem gebunden sind.

Abgesehen von den primären und gegebenenfalls sekundären Aminogruppen, tertiären Aminogruppen oder Hydroxylgruppen enthalten die Co-Härter ansonsten vorzugsweise keine weiteren funktionellen Gruppen.

Bevorzugte Co-Härter sind z.B. aliphatische Aminverbindungen, welche außer einer primären Aminogruppe keine weiteren funktionellen Gruppen enthalten, z.B.C2- bis C8-Alkylenamine, wie Ethylamin, Propylamin oder Butylamin.

Bevorzugte Co-Härter sind z.B.auch lineare oder verzweigte aliphatische Aminverbindungen, welche zwei primäre Aminogruppen und ansonsten keine weiteren funktionellen Gruppen enthalten, z.B. C2- bis C8-Alkylendiamine, wie Ethylendiamin, Propylendiamin oder Butylendiamin.

Bevorzugte Co-Härter sind z.B. auch aliphatische Aminverbindungen, welche eine oder zwei primäre Aminogruppen und ein oder zwei Hydroxylgruppen und ansonsten keine weiteren funktionellen Gruppen enthalten, z.B.Monoamine, wie C2- bis C8- Alkanolamine, wie Ethanolamin, Isopropanolamin,

Bevorzugte Co-Härter sind z.B.auch aliphatische Aminverbindungen, welche eine primäre Aminogruppe und eine tertiäre Aminogruppe ansonsten keine weiteren funktionellen Gruppen enthalten. Genannt seien z.B.Verbindungen der Formel

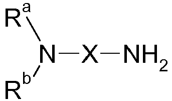

In der vorstehenden Formel stehen Ra und Rb unabhängig voneinander für eine C1- bis C10-, vorzugsweise eine C1- bis C4- Alkylgruppe. X steht für eine C2- bis C10-, vorzugsweise eine C2- bis C4-Alkylengruppe. Die Alkylengruppe kann verzweigt oder linear sein; sie ist an beliebiger Stelle durch die tertiäre und die primäre Aminogruppe substituiert. In einer bevorzugten Ausführungsform ist die Alkylengruppe linear und endständig durch die tertiäre und primäre Aminogruppe substituiert. Als eine der hier besonders bevorzugten Co-Härter sei z.B. 3-Dimethylamino-propylamin (DMAPA) genannt.

Bevorzugte Co-Härter sind auch aliphatische Aminverbindungen, welche eine oder zwei primäre Aminogruppen, vorzugsweise eine primäre Aminogruppe, und eine sekundäre Aminogruppe und eine Hydroxylgruppe und ansonsten keine weiteren funktionellen Gruppen enthalten. Hierbei handelt es sich insbesondere um N-(2-Aminoalkyl)alkanolamine, z.B.N-(2-Aminoethyl)-ethanolamin (H2N-CH2-CH2-NH-CH2-CH2-OH). Bevorzugt haben die bestehen die beiden Alkylengruppen in diesen Verbindungen aus 2 bis 8 C-Atomen.

Bevorzugte aromatische Co-Härter sind z.B. auch durch ein bis drei Aminomethylengruppen (H2N-CH2-) substituiertes Benzol. Insbesondere handelt es sich um Benzol, welches durch zwei H2N-CH2- Gruppen an beliebiger Position des Benzolrings substituiert ist, z.B. um Xylendiamin mit der Formel

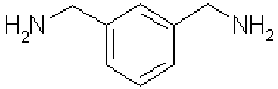

Bevorzugte cycloaliphatische Co-Härter sind z.B. auch durch ein bis drei Aminomethylengruppen (H2N-CH2-) substituiertes Cyclohexan. Insbesondere handelt es sich um Cyclohexan, welches durch zwei H2N-CH2- Gruppen an beliebiger Position des Benzolrings substituiert ist.

In Betracht kommen natürlich auch beliebige Mischungen der vorstehenden Co-Härter.

Die Co-Härter haben vorzugsweise ein Molekulargewicht kleiner 500 g/mol, insbesondere kleiner 300 g/mol.

Bevorzugte Co-Härter bestehen insgesamt aus maximal 10 C-Atomen, besonders bevorzugte Co-Härter bestehen insgesamt aus maximal 8 C-Atomen.

Von den oben genannten Co-Härtern sind die aliphatischen Verbindungen bevorzugt; besonders bevorzugte aliphatischen Verbindungen sind solche mit nur einer primären Aminogruppe und gegebenenfalls einer tertiären Aminogruppe oder gegebenenfalls einer Hydroxylgruppe und ansonsten keiner weiteren funktionellen Gruppe.

Vorzugsweise ist der Gewichtsanteil der Co-Härter von 2 bis 40 Gew. %, besonders bevorzugt von 5 bis 35 Gew. %, bezogen auf die Gewichtssumme aller Amino-Härter.

Die Co-Härter werden vorzugsweise in Mengen von 0,1 bis 30 Gew. teilen, besonders bevorzugt in Mengen von 0,5 bis 20 Gewichtsteilen, bezogen auf Epoxyverbindungen eingesetzt.

Bei den neben den Co-Härtern eingesetzten Amino-Härtern handelt es sich um Amino-Härter H2, die nicht unter die vorstehende Definition der Co-Härter fallen, wie oben ausgeführt. Der Anteil dieser Amino-Härter H2 ist dann entsprechend vorzugsweise 60 bis 98 Gew. %, besonders bevorzugt 65 bis 95 Gew. %, bezogen auf die Gewichtssumme aller Amin-Härter.

Derartige Aminohärter H2 sind z.B. Polyamidoamine, Phenalkamine, Epoxy-Aminaddukten, Polyetheraminen oder sonstige von den Aminohärtern H1 (Co-Härtern) verschiedene Aminoverbindungen oder deren Gemische.

Bevorzugt handelt es sich bei den Aminohärtern H2 um Polyamidoamine, Phenalkamine, Epoxy-Aminaddukte, Polyetheramine oder deren Gemische.

Wenn Gemische von verschiedenen Aminohärtern verwendet werden, können sie vorab gemischt und der Epoxidharz-zusammensetzung dann als Mischung zugesetzt werden, sie können jedoch auch separat zugesetzt werden. Sie können auch gleichzeitig oder in Verbindung mit anderen Bestandteilen der Epoxidharz-zusammensetzung zugegeben werden. Als derartige Bestandteile kommen z.B. die oben genannten Additive in Betracht.

Die zur Aushärtung benötigte Gesamtmenge an Amino-Härter, bzw. Gewichtssumme aller Amino-Härter H1 und H2, bestimmt sich in an sich bekannter Weise über die Anzahl der Epoxidgruppen in der Formulierung und die Anzahl der funktionellen Gruppen im Härter. Die Anzahl der Epoxidgruppen im Epoxidharz wird als sogenanntes Epoxidäquivalent angegeben. Das Epoxidäquivalent wird gemäß DIN 16945 bestimmt.

Die Anzahl der primären und sekundären Aminogruppen kann über die Aminzahl gemäß DIN 16945 berechnet werden.

Die Amino-Härter werden vorzugsweise insgesamt in solchen Mengen eingesetzt, dass das Verhältnis der Anzahl aller primären und sekundären Aminogruppen und der Anzahl aller Epoxidgruppen im Epoxidharz 2 : 1 bis 1 : 2, vorzugsweise 1,5 : 1 bis 1 : 1,5 und insbesondere 1,1 : 1 bis 1 : 1,1 beträgt. Bei einem stöchiometrischen Verhältnis von etwa 1 : 1 erhält man ein gehärtetes Harz mit optimalen duroplastischen Eigenschaften. Je nach gewünschten Eigenschaften des Harzes nach Vernetzung kann es aber auch sinnvoll sein, Härter und Epoxidharz in anderen Verhältnissen der reaktiven Gruppen einzusetzen.

In den Epoxidharz-zusammensetzungen beträgt dementsprechend die Gesamtmenge an Amino-Härtern (Gewichtssumme aus H1 und H2) in der Regel 0,1 Gew.-% bis 50 Gew.-%, häufig 0,5 bis 40 Gew.-% und insbesondere 1 bis 30 Gew.-%, bezogen auf die Gewichtssumme aus Epoxyverbindungen, Verbindungen der Formel I, Aminohärter H1 und H2.

Neben Amino-Härtern können auch andere Härter, z.B. Anhydrid-Härter, mitverwendet werden. In einer bevorzugten Ausführungsform werden jedoch ausschließlich Verbindungen Amino-Härter verwendet.

### Zu weiteren Bestandteilen der Epoxidharz-zusammensetzung

Neben den Epoxidharzen und den Verbindungen der Formel I können die Epoxidharzzusammensetzungen noch sonstige Verdünnungsmittel, auch sonstige Reaktivverdünner enthalten.

Sonstige Reaktivverdünner sind z.B.niedermolekulare Verbindungen mit einem Molekulargewicht von vorzugsweise maximal 250 Dalton, z.B.im Bereich von 100 bis 250 Dalton, die Oxirangruppen, vorzugsweise Glycidylgruppen, z.B.in Form von Glycidylethergruppen, Gylcidylestergruppen oder Glycidylamidgruppen, aufweisen.

Als sonstige Reaktivverdünner genannt seinen z.B. Glycidylether von gesättigten Alkanolen mit 2 bis 20 C-Atomen, oder alkoxylierten Alkanolen.

In einer besonderen Ausführungsform enthalten die Epoxidharz-zusammensetzungen keine anderen Reaktivverdünner als Die obigen Verbindungen der Formel I. Soweit andere Reaktivverdünner mitverwendet werden, kann das Gewichtsverhältnis von Verbindung der Formel I zu anderen Reaktivverdünnern zum Beispiel im Bereich von 1:10 bis 10:1, insbesondere im Bereich von 1:5 bis 5:1 liegen

Die Epoxidharz-zusammensetzungen können darüber hinaus auch nicht-reaktive, organische Verdünnungsmittel enthalten. Hierunter versteht man organische Lösungsmittel, die bei Normaldruck einen Siedepunkt unterhalb 200°C aufweisen und die mit den Epoxidgruppen und den Gruppen eines gegebenenfalls vorhandenen Reaktivverdünners keine Reaktion unter Bindungsbildung eingehen und bei der späteren Verwendung entweichen. Exemplarisch genannt seien aliphatische, cycloaliphatische oder aromatische Kohlenwasserstoffe, Alkohole, Ketone oder Ester.

In einer bevorzugten Ausführungsform enthält die Epoxidharz-zusammensetzungen nicht reaktive organische Lösemittel allenfalls in untergeordneten Mengen (weniger als 20 Gew.-%, insbesondere weniger als 10 Gew.-% oder weniger als 5 Gew.-%, bezogen auf die Gewichtssumme aus Epoxidharz und Verbindung der Formel I) und besonders bevorzugt enthalten sie kein derartiges Lösemittel (100 % System).

Neben den vorgenannten Bestandteilen kann die Epoxidharz-zusammensetzung Füllstoffe und/oder sonstige Additive enthalten, welche für die jeweilige Verwendung der Epoxidharz-zusammensetzungen gewünscht sind.

Geeignete Füllstoffe sind beispielsweise anorganische oder organische partikelförmige Materialien wie beispielsweise Calciumcarbonate und Silikate sowie anorganische Fasermaterialien wie beispielsweise Glasfaser. Auch organische Füllstoffe wie Kohlefaser und Mischungen aus organischen und anorganischen Füllstoffen, wie beispielsweise Mischungen aus Glas- und Kohlefasern oder Mischungen aus Kohlefasern und anorganischen Füllstoffen können Anwendung finden. Die Füllstoffe können zum Beispiel in einer Menge von 1 bis 70 Gew.-%, bezogen auf das Gesamtgewicht der Epoxidharz-zusammensetzung, zugesetzt werden.

Geeignete herkömmliche Additive umfassen beispielsweise Antioxidantien, UV-Absorber/ Lichtstabilisatoren, Metalldeaktivatoren, Antistatika, Verstärkungsstoffe, Füllstoffe, Antifoggingmittel, Treibmittel, Biozide, Weichmacher, Gleitmittel, Emulgatoren, Farbmittel, Pigmente, Rheologiemittel, Schlagzähigkeitsverbesserer, Katalysatoren, Adhäsionsregulatoren, optische Aufheller, Flammschutzmittel, Antitropfmittel, Nukleierungsmittel, Verlaufsmittel, Entschäumer,

Als weitere Bestandteile der Epxidharz-zusammensetzungen, bzw. einer separaten Härtermischung, kommen auch Katalysatoren in Betracht, welche die Härtungsreaktion beschleunigen, z.B. Phosphoniumsalze organischer oder anorganischer Säuren, Imidazol und Imidazolderivate, tertiäre Aminoverbindungen wie Triethanolamin oder Triisopropanolamin oder quartäre Ammoniumverbindungen. Derartige andere Katalysatoren werden, sofern erwünscht, in Anteilen von 0,01 Gew.-% bis etwa 10 Gew.-%, bezogen auf das Gesamtgewicht des Epoxidharzes, der Verbindung I und Gesamtmenge der Aminohärter eingesetzt. In einer bevorzugten Ausgestaltung werden keine derartigen Katalysatoren benötigt, d. h. der Gehalt an derartigen Katalysatoren in der Zusammensetzung beträgt weniger als 0,5, insbesondere weniger als 0,1 Gew.-% bzw. weniger als 0,01 Gew. %.

### Zur Verwendung der Epoxidharz-zusammensetzungen

Bei Epoxidharz-zusammensetzungen unterscheidet man grundsätzlich zwischen einkomponentigen- (1K) und zweikomponentigen- (2K) Bindemittel-systemen. Bei 2K-Systemen bleiben die Epoxyverbindungen und Härter bis kurz vor der Härtung getrennt (daher 2K), da das Epoxidharz und der Härter sehr reaktiv sind. Bei 2K-Systemen wird der Härter erst kurz vor der Härtung zu der Epoxyverbindung, bzw. zu der Epoxidharz-zusammensetzung, gegeben.

Die erfindungsgemäße Epoxidharz-zusammensetzungen sind insbesondere 2K-Systeme.

Die Zugabe der Härter, vorzugsweise einer Härtermischung, welche mindestens einen Härter, vorzugsweise mindestens einen Aminohärter enthält, erfolgt entsprechend erst kurz vor der Verwendung.

Die zweikomponentige Epoxidharz-zusammensetzung umfasst daher eine separate Epoxidharz-zusammensetzung, welche Epoxidharze, Verbindungen der Formel I und gegebenenfalls weitere Bestandteile, jedoch keine Härter, insbesondere keinen Aminohärter, enthält, und eine separate Härtermischung, welche Härter, vorzugsweise Aminohärter, jedoch keine Epoxyverbindungen enthält.

Bei den 2 K Systemen handelt es sich daher um ein System aus einer Komponente A, enthaltend
a) Verbindungen der Formel (I) und
b) Verbindungen mit mindestens einer Epoxygruppe (kurz Epoxyverbindungen) und
   einer Komponenten B, enthaltend
c) Härter für Epoxyverbindungen.

Sowohl A als auch B können weitere Bestandteile enthalten, insbesondere die oben genannten weiteren Bestandteile.

Die Härtermischung enthält besonders bevorzugt eine Mischung von Aminohärtern H1 und H2 und gegebenenfalls weitere Bestandteile, z.B.Katalysatoren (siehe oben).

Nach Zugabe der Härter zur Epoxidharz-zusammensetzung erfolgt die Härtung.

Die Härtung kann dann thermisch durch Erwärmen der Zusammensetzung erfolgen. Üblicherweise erfolgt die Härtung der Epoxidharz-zusammensetzungen bei Temperaturen im Bereich von -10 bis 200°C, vorzugsweise im Bereich von -10 bis 180°C und insbesondere im Bereich von -10 bis 150°C.

Alternativ kann die Härtung z.B.auch mikrowelleninduziert erfolgen. Insbesondere erfolgt die Härtung bei -10 bis 80°C und in einer besonders bevorzugten Ausführungsform bei -10 bis 40°C bzw. bei -10 bis 20°C. Vorteilhaft ist, dass die Härtung unter normalen Umgebungsbedingungen wie Raumtemperatur und/oder Einwirkung von Sonnenlicht erfolgen kann.

Die Epoxidharz-zusammensetzungen als 1K- oder als 2K-system in verschiedensten technischen Anwendungen eingesetzt werden. unabhängig davon, ob es sich um 1K- oder 2K-Systeme handelt.

Sie eignen sich beispielsweise als Beschichtungsmassen, Gießmassen, zur Herstellung von Verbundwerkstoffen, als Klebstoffe, insbesondere Strukturklebstoff, oder zur Imprägnierung von Fasern oder Fasergeweben. Sie können in diesen Verwendungen als alleinige Bindemittel oder auch in Kombination mit anderen Bindemitteln verwendet werden.

Als Beschichtungsmassen seien z.B.Lacke genannt. Mit den erfindungsgemäßen Epoxidharz-Zusammensetzungen können z.B. kratzfeste Schutzlacke auf beliebigen Substraten, z.B.aus Metall, Kunststoff oder Holzwerkstoffen erhalten werden.

Da die Reaktivität der Epoxidharz-zusammensetzungen vergleichsweise hoch ist, kann die Härtung bei niedrigen Temperaturen, z.B.im Bereich von 0 bis 50°C und insbesondere im Bereich von 5 bis 35°C erfolgen. Dies macht die Epoxidharz-zusammensetzungen in besonderer Weise für die Beschichtung sehr großflächiger Substrate geeignet, die sich nicht oder nur schwierig auf Temperaturen oberhalb der Umgebungstemperatur erwärmen lassen. Hierzu zählt insbesondere die Beschichtung von Böden, insbesondere in stark beanspruchten Bereichen, z.B.zur Beschichtung von Laufbereichen öffentlicher Gebäude oder Plätze oder zur Beschichtung von Parkflächen und Zufahrten von Parkflächen. Hierzu zählt insbesondere auch die Beschichtung großflächiger Metallteile und Metallkonstruktionen, beispielsweise in oder an Gebäuden oder Schiffen (sog. Marine Coating).

Die Epoxidharz-zusammensetzungen eignen sich auch als Isolierbeschichtungen in elektronischen Anwendungen, z.B.als Isolierbeschichtung für Drähte und Kabel. Genannt sei auch die Verwendung zur Herstellung von Photoresisten. Sie eignen sich insbesondere auch als Reparaturlack, z.B.auch bei der Ausbesserung von Rohren ohne Demontage der Rohre (cure in place pipe (CIPP) rehabilitation). Sie eignen sich auch zur Versiegelung bzw. Beschichtung von Fußböden.

Die Epoxidharz-zusammensetzungen können als Gießmassen zur Einbettung, Anbindung oder Verfestigung von Formkörpern, z.B.in elektronischen Anwendungen, eingesetzt werden. Sie eignen sich als Flip-chip underfill oder als Elektrogießharze für potting, casting und (glob-top-) encapsulation.

Die Epoxidharz-zusammensetzungen eigen sich insbesondere auch zur Herstellung von Verbundwerkstoffen. In Verbundwerkstoffen (Composites) sind unterschiedliche Materialien, z.B.Kunststoffe und Verstärkungsmaterialien (Fasern, Carbonfasern) durch das ausgehärtete Epoxidharz miteinander verbunden.

Die Epoxidharz-zusammensetzungen eignen sich auch als Klebstoffe, insbesondere Strukturklebstoffe. Strukturklebstoffe dienen zur dauerhaften Verbindung von Formteilen miteinander. Die Formteile können aus beliebigem Material sein; in Betracht kommen Materialien aus Kunststoff, Metall, Holz, Leder, Keramik etc. Es kann sich dabei auch um Schmelzklebstoffe (hot melt adhesives) handeln, die erst bei höherer Temperatur fließfähig und verarbeitbar sind. Es kann sich auch um Fußbodenklebstoffe handeln. Die Zusammensetzungen eignen sich auch als Klebstoffe für die Herstellung von Leiterplatten (electronic curcuits), insbesondere auch nach der SMT Methode (surface mounted technology).

Die Epoxidharz-zusammensetzungen eignen sich z.B.zur Herstellung von imprägnierten Fasern oder zur Herstellung von aus Fasern hergestellten vorimprägnierter Garne und Gewebe, z.B.zur Herstellung von Prepregs die zu Verbundwerkstoffen weiterverarbeitet werden. Als Herstellverfahren für Verbundwerkstoffe seien die Härtung von vorimprägnierten Fasern oder Fasergeweben (z.B.Prepregs) nach Lagerung oder aber die Extrusion, Strangziehen (pultrusion), Wickeln (winding) und Resin Transfer Molding (RTM), Resin Infusion Technologies (RI) genannt. Insbesondere können die Fasern bzw. die daraus hergestellten Garne und Gewebe mit der erfindungsgemäßen Zusammensetzung getränkt werden und danach bei einer höheren Temperatur gehärtet werden.

Insbesondere eignet sich die Epoxidharz-zusammensetzung für Verfahren, z. B der Beschichtung von Oberflächen, Imprägnierung, Formgebung oder ähnliches, bei dem man die Epoxidharz-Zusammensetzung auf die zu beschichtende Oberfläche aufbringt bzw. in die gewünschte Form bringt und anschließend aushärtet. Diese Verfahren unterliegen in Hinblick auf die zu beschichtende Oberfläche keinen Beschränkungen. Beispiele für geeignete Oberflächen sind Metalloberflächen, Holzoberflächen, Glasoberflächen, Plastikoberflächen..

Verbindungen der Formel I sind nach den oben beschriebenen Herstellungsverfahren leicht und in guter Ausbeute erhältlich. Sie eigenen sich z.B. sehr gut als Reaktivverdünner in Epoxidharzzusammensetzungen.

Als besondere Vorteile der Epoxidharz-zusammensetzungen seien die geringe Viskosität durch Mitverwendung von Verbindungen der Verbindungen der Formel I und die guten anwendungstechnischen Eigenschaften und hier insbesondere die sehr gute Elastizität genannt. Verbindungen der Formel I klönnen in den Epoxidharz-Zusammensetzungen in geringen aber auch in großen Mengen verwendet werden.

### Beispiele

### 1. Materialien

### ExoVC-acrylat 1:

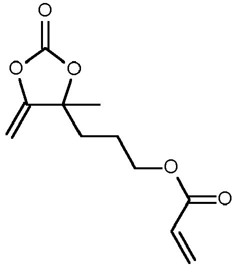

### ExoVC-acrylat 2:

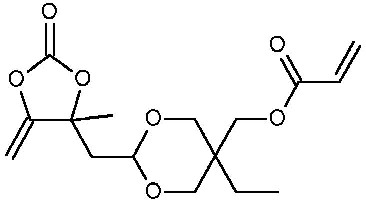

### ExoVC, zum Vergleich

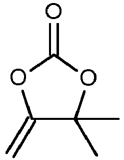

### 4,4-Dimethyl-5-methylene-1,3-dioxolan-2-one (kurz ExoVC) gemäß WO2011/157671

### Epoxidharz:

Aromatisches Epoxidharz auf Basis von Bisphenol A mit einem Epoxid-Äquivalent von 175 - 185 g/Äquiv. und einer Viskosität bei 25 °C von 8 - 10 PA.s (Epilox A 18-00)

**Härter**

| | |
|---|---|
| Härter H1: | Polyetheramine D230 (BASF SE) |
| Härter H2: | Isophorondiamine (IPDA, BASF SE) |

### 2. Herstellungsbeispiele

### 2.1. Herstellung des ExoVC-acrylat 1

Die Herstellung erfolgt in vier Stufen.

Das nachstehende Reaktionsschema umfasst die Stufen 1 bis 3

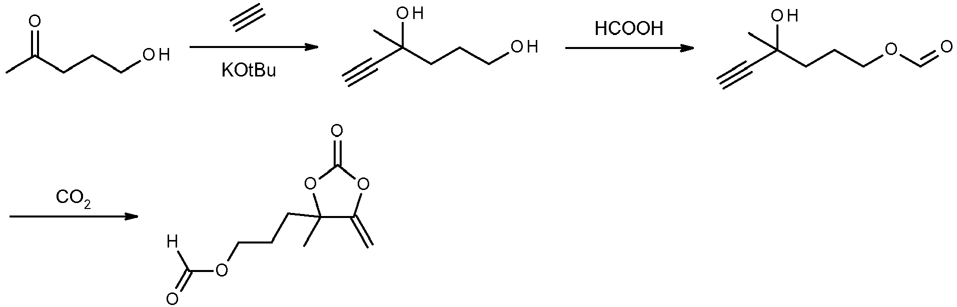

### 1. Stufe: Umsetzung von Hydroxypentanon mit Acetylen zum Acetylen-Addukt:

KOtBu (800 g, 7,1 mol) wird in wasserfreiem THF (4,5 L) vorgelegt und auf 0-3°C gekühlt. Acetylen (280 g, 10,8 mol) wird innerhalb von 3 h bei dieser Temperatur eingeleitet. Unter weiterem Einleiten von Acetylen (130 g, 5 mol) wird dann Hydroxypentanon (550 g, 5.39 mol) innerhalb von 1 h bei 0-6°C zugetropft. Hierbei entsteht nach Durchlaufen eines Viskositätsmaximums eine orangebraune Lösung.

Nach 1 h Nachrühren bei 0-3 °C wird auf RT erwärmt und bei 20-25°C Ammoniumchlorid (1068 g in 5 L Wasser) innerhalb von 45 min zugegeben. Dabei bilden sich zwei Phasen.
Die organische Phase wird abgetrennt, über Natriumsulfat getrocknet und das Lösemittel wird bei 40 °C und 5 mbar am Rotationsverdampfer entfernt.
Es werden 649 g eines braunen Öls erhalten. Dieses wird im Ölpumpen-Vakuum destilliert, wobei auf eine Sumpftemperatur von max. 130°C geachtet wurde. Die Hauptfraktion wurde bei 110-111 °C in Form eines gelben Öls (440 g, 3,4 mol, 64%) erhalten.
Reinheit (GC-Flächen-%): 93%

### 2. Stufe: Umsetzung des Diols mit Ameisensäure zum Formiat:

Das in der ersten Stufe erhaltene Diol (1.305 kg, 10.2 mol) wird vorgelegt und innerhalb von 1 h bei 7-8 °C mit Ameisensäure (1.512 L, 40 mol) versetzt. Dann wird auf Raumtemperatur erwärmt und man beobachtet das Fortschreiten der Reaktion mittels GC/FID. Man lässt die Reaktion so lange laufen, bis der Hauptpeak praktisch nicht mehr zunimmt, was nach ca. 4 h bei RT der Fall ist. Führt man die Reaktion über diesen Punkt hinaus fort, bildet sich vermehrt zweifach formyliertes Produkt.

Zur Aufarbeitung wird zunächst im Vakuum, ohne zu erwärmen, eingeengt und das erhaltene Rohprodukt dann bei 12 mbar destilliert. Die Hauptfraktion geht bei 112-115 °C als klare, gelbe Flüssigkeit (1.453 kg, 9.3 mol, 91%) über.
Reinheit (GC-Flächen-%): 93%

### 3. Stufe: Ringschluss mit CO₂ zum ExoVC-formiat

Das in der zweiten Stufe erhaltene Formiat (1.623 kg, 10.4 mol) wird mit Silberacetat (1.38 g) und TMTACN (N,N',N"-trimethyl-1,4,7-triazacyclononan, 13.8 mL) versetzt und in einem Rührautoklaven mit Druckhalteventil mit 10 bar CO₂ beaufschlagt.
Innerhalb von 5 h steigen die Temperatur auf 56°C und der Druck auf 18 bar an.
Wenn die Temperatur wieder auf RT abgefallen ist, wird auf 70 °C erwärmt. Der Druck steigt dabei auf ca. 28 bar an. Nach ca. 4 h zeigt die Reaktionskontrolle praktisch kein Edukt mehr (GC/FID).

Es wird abgekühlt und entspannt. Der Autoklaveninhalt wird nach Zusatz von Dichlormethan (1.5 L) zweimal mit HCl (10%ige Lösung, je 480 mL) gewaschen, über Natriumsulfat getrocknet und das Lösemittel am Rotationsverdampfer entfernt (40°C, 60 mbar).
Danach werden unter Rühren bei RT alle im Ölpumpenvakuum flüchtigen Bestandteile entfernt, was bis zu 48 h dauern kann. Es werden ca. 1,9 kg Rohprodukt erhalten.
Zur weiteren Aufreinigung werden in einem Dünnschichtverdampfer mit Wischersystem zunächst flüchtige Komponenten abgetrennt (0,05-0,02 mbar, Manteltemperatur 109-114°C). Hierfür sind in der Regel mindestens zwei Läufe erforderlich.
In einem weiteren Lauf wird dann die Zielverbindung von schwerflüchtigen Bestandteilen abgetrennt (0,012 mbar, Manteltemperatur 170°C).
Ausbeute an ExoVC-formiat: 1,419 kg (7,1 mol, 68%).
Reinheit (GC-Flächen-%): 97%

### 4. Stufe: Acrylierung zum ExoVC-acrylat 1

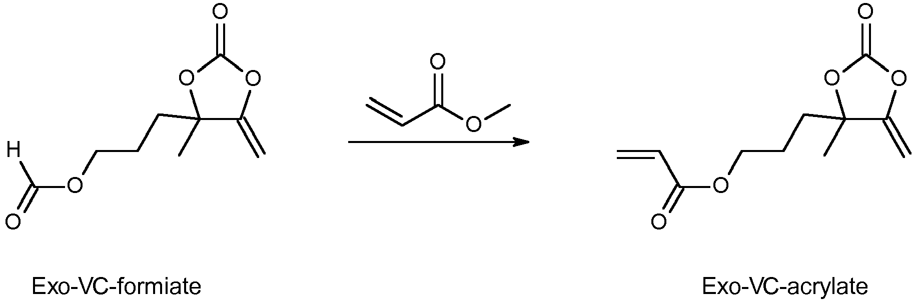

In einem zwei Liter Flachkolben mit Teflonrührer, Thermometer und Kühler wurden 300g Exo-VC-formiat (1.5 mol) und 1200 g Methylacrylat (14 mol) gegeben. Das Methylacrylat enthielt 0,3g MeHQ (Monomethylether des Hydrochinons) als Inhibitor. Die Mischung wurde gerührt und auf 40°C erwärmt. 30 g des Enzyms Candida Antartica Lipase B (immobilisierte Form) wurden zugesetzt.

Nach 24 Stunden wurde die erhaltene Mischung in einen zwei Liter Rundkolben dekantiert, wobei das Enzym in dem Flachkolben zurückblieb. Das entstandene Methanol und gleichzeitig Methylacrylat wurden unter Vakuum entfernt.

Der Rückstand wurde zusammen mit 1000g frischem Methylacrylat zurück in den Flachkolben gegeben, der das Enzym enthält. Die erhaltene Mischung wurde erneut 24 Stunden bei 40°C gerührt.
Die Reaktionsmischung wurde auf Raumtemperatur abgekühlt und filtriert. Methanol und nicht umgesetztes Methylacrylat wurden unter Vacuum abdestilliert.
Die Ausbeute betrug 339 g Exo-VC-acrylat 1
Reinheit: 96.7% (gaschromatographisch bestimmt)

### 2.2 Herstellung von ExoVC-acrylat 2

Die Herstellung erfolgt in vier Stufen.

Das nachstehende Reaktionsschema umfasst die Stufen 1 bis 3

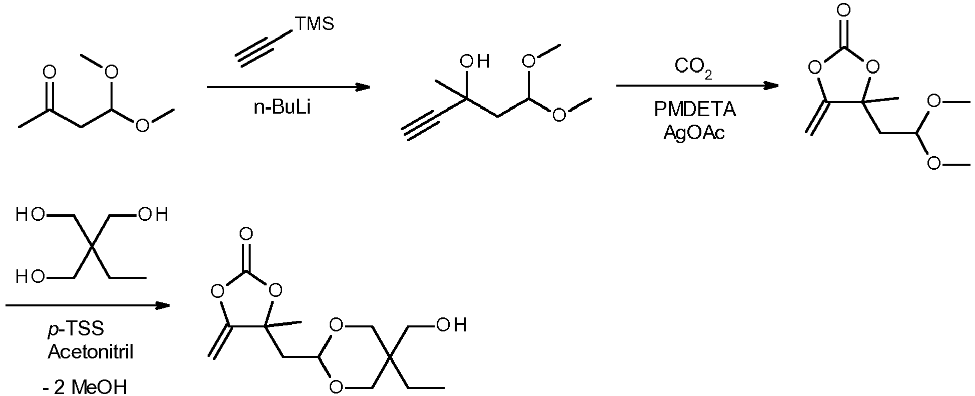

### 1.) Ethinylierung von 4,4-Dimethoxybutan-2-on:

TMS-Acetylen (982 g, 10 mol) wird in THF (17 L, getrocknet über Molsieb) unter Argon vorgelegt und auf -68 °C abgekühlt. Unter Rühren wird innerhalb von 1 h *n*-Butyllithium (2,5 M in Hexan, 4 L) bei -68 °C zugetropft und 1 h nachgerührt.

Innerhalb von 30 min wird dann das Keton (1,319 kg, 10 mol) bei -68°C bis -54°C zugetropft und in Anschluss 15 min nachgerührt. Danach wird auf 9°C erwärmt und Wasser (2,9 L) wird in einer Portion zugegeben. Dabei steigt die Temperatur auf ca. 17°C an.

Das Reaktionsgemisch wird bei 45°C/8 Torr gründlich eingeengt. Durch GC-Analytik wird sichergestellt, dass kein TMS-geschütztes Produkt mehr enthalten ist.

Der Rückstand wird in Diethylether (750 mL) aufgeschlämmt, filtriert und der Filtrationsrückstand nochmals mit Diethylether gewaschen. Das Filtrat wird im Vakuum eingeengt. Es bleiben ca. 1,2 kg Rohmaterial als braune Flüssigkeit.

Durch Vakuumdestillation (5 mbar) werden daraus bei 64-68 °C ca. 1,1 kg (7 mol, 70%) ethinyliertes Produkt als farbloses Öl erhalten.
Reinheit: >96% (GC-Flächen%)

### 2.) Ringschluss mit CO₂:

Der in Stufe 1 erhaltene Acetylenalkohol (1233 g; 7,79 mol) wird in Acetonitril (1,2 L) vorgelegt und in einem Rührautoklaven mit PMDETA (Pentamethyldiethylentriamin; 138,9 g; 0,8 mol) und AgOAc (12,9 g; 0,078 mol) versetzt. Es werden 50 bar CO₂ aufgepresst und 2,5 h gerührt. Die Temperatur steigt bis auf 75°C.

Das Reaktionsgemisch wird nach Abkühlen auf Raumtemperatur auf Normaldruck entspannt, filtriert und bei 100°C/5 mbar eingeengt. Es bleiben ca. 1,5 kg Rohmaterial als braune Flüssigkeit.

Durch Vakuumdestillation bei 5 mbar werden daraus bei 114-115°C ca. 1,39 kg des Carbonats als orangefarbenes Öl erhalten, welches (ggfs. Nach Zusatz einiger Impfkristalle) über Nacht durchkristallisiert.
Die Kristallmasse wird mit Cyclohexan (1,34 L) verrührt, abgesaugt und der Rückstand nochmals mit Cyclohexan (0,45 L) nachgewaschen.

Nach Trocknen im Vakuum werden 1,29 kg (6,38 mol, 64%) fast farbloser Feststoff erhalten.
Reinheit: >99% (GC-Flächen%)

### 3.) Umacetalisierung mit Trimethylolpropan:

Das dimethoxy-substituierte Carbonat aus Stufe 2 (250 g, 1,24 mol) wird in 1,4 L Acetonitril unter Argonatmosphäre vorgelegt. Dann werden 253 g (1,87 mol) Trimethylolpropan und 407 mg (0,002 mol) *p*-Toluolsulfonsäure-Hydrat zugegeben. Die Mischung wird 10 h unter Rückfluss erhitzt.

Nach Abkühlen auf Raumtemperatur wird das Lösemittel im Vakuum entfernt und der Rückstand in ca. 1 L MTBE aufgenommen. Es wird viermal mit je 300 mL Wasser gewaschen. Die organische Phase wird über Natriumsulfat getrocknet, abfiltriert und am Rotationsverdampfer eingeengt. Anschließend wird bei 40°C im Ölpumpenvakuum für mehrere Stunden nachgetrocknet.

Das Produkt wird als zähflüssiges, leicht gelbliches Öl (339 g) in Form zweier Isomere erhalten, welches bei längerem Stehen bei Raumtemperatur langsam kristallisiert.
Reinheit: >98% (GC-Flächen%)

### 4.) Acrylierung zum ExoVC-acrylat 2

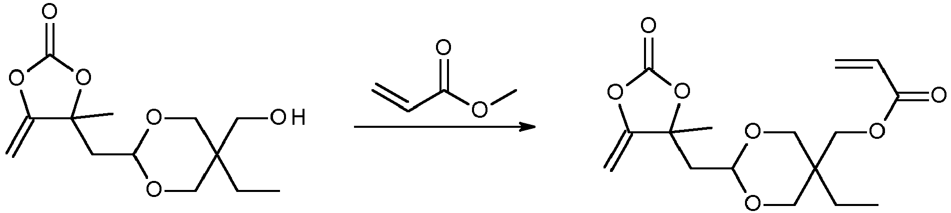

In einem zwei Liter Flachkolben mit Teflonrührer, Thermometer und Kühler wurden 129 g Exo-VC-PMP Alcohol (0.47 mol), 409 g Methylacrylat (4.75 mol) und 152 g Molekularsieb 5 Ǻ Pulver gegeben. Das Methylacrylat enthielt 0,3 g MeHQ (Monomethylether des Hydrochinons) als Inhibitor. Die Mischung wurde gerührt und auf 60°C erwärmt. 9,7 g des Enzyms Candida Antartica Lipase B (immobilisierte Form) wurden zugesetzt.

Nach 72 Stunden wurden 100 g Molekularsieb 5 Ǻ Pulver und 9,7 g des Enzyms Candida Antartica Lipase B (immobilisierte Form) zugesetzt.
Nach 96 Stunden wurde die Reaktionsmischung auf Raumtemperatur abgekühlt und filtriert. Methanol und nicht umgesetztes Methylacrylat wurden unter Vacuum abdestilliert.
Die Ausbeute betrug 153 g ExoVC-acrylat 2.
Reinheit: 97.1% (gaschromatographisch bestimmt)

### 3. Anwendungstechnische Prüfungen

Die Epoxidharz-zusammensetzungen wurden durch Mischen der Epoxidharze, der Härter und der Reaktivverdünner (ExoVC acrylat 1, ExoVC acrylat 2 oder ExoVC) hergestellt. In den Tabellen finden sich die Angaben zu den verwendeten Epoxidharzen, Härtern und Reaktivverdünnern sowie deren Gewichtsteile.

### 3.1 Viskosität und Reaktivität

Die Anfangsviskosität der Epoxidharz-zusammensetzungen wurde bei 25°C mit einem Platte/Platte Viskosimeter mit einer Spaltweite von 1mm bestimmt (MCR302, Anton Paar).

Mit dem gleichen Gerät wurde der Anstieg der Viskosität der Zusammensetzungen über die Zeit gemessen (Temperatur 25°C). In den Tabellen ist die Zeit bis zur Verdopplung der Anfangsviskosität angegeben. Je kürzer die Zeit, desto höher ist die Reaktivität.

### 3.2 Glasübergangstemperatur Tg

Die Glasübergangstemperaur T_{g} der Epoxidharz-zusammensetzungen wurde durch DSC (Differential Scanning Calorimetry) gemäß ASTM 3418/82 bestimmt.

### 3.3 Tiefungsgprüfung (DIN EN ISO1520)

Mit der Tiefungsprüfung wird die Elastizität von Beschichtungen auf metallischem Untergrund bestimmt. Ein Stahlblech wurde mit den Epoxidharz-zusammensetzungen (Schichtdicke 100 µm) beschichtet. Das beschichtete Blech wurde über Nacht bei Raumtemperatur gelagert. In einer Vorrichtung für die Durchführung der Tiefungsprüfung wurde das Blech durch Aufdrücken eines Stempels auf die nicht beschichtete Seite verformt, so dass sich die beschichtete Seite entsprechend ausbeult. Die Höhe der Ausbeulung bei der zum ersten Mal Risse in der Beschichtung festgestellt werden, ist ein Maß für die Elastizität. Je höher die Ausbeulung bei den ersten Rissen, desto elastischer ist die Beschichtung. Die in den Tabellen angegebenen Werte sind Mittelwerte aus drei Messungen.

### 3.4 Shore D -Härte

Die Epoxidharz-zusammensetzungen wurden in einer Menge von 10 g in Schälchen mit einem Radius von 4 cm gegeben und 7 Tage bei 25°C gelagert. Bei der Bestimmung der Shore-D Härte wird eine definierte Nadel mit definierter Länge vollständig in die Beschichtung gedrückt. Die Shore D-Härte entspricht der Kraft, die dazu notwendig ist. In den Tabellen sind die Mittelwerte aus 5 Messungen angegeben. Je größer die Kraft desto höher ist die Härte.

**Tabelle1:**

| Anfangsviskosität und Reaktivität | | | | | | | |
|---|---|---|---|---|---|---|---|
| Nr. | Epoxidharz | Verdünner | | Härter | | Anfangs viskosität | Reaktivität |
| | Gew.-teile | Typ | Gew.-teile | Typ | Gew.-teile | mPas | min |
| 1 | 10 | -- | 0 | H1 | 3,4 | 577 | 180 |
| 2 | 9 | ExoVC acrylat1 | 1 | H1 | 4,0 | 340 | 28 |
| 3 | 7 | ExoVC acrylat1 | 3 | H1 | 5,3 | 310 | 9,8 |
| 4 | 5 | ExoVC acrylat1 | 5 | H1 | 6,8 | 125 | 4,2 |
| 5 | 3 | ExoVC acrylat1 | 7 | H1 | 8,0 | 80 | 3 |
| 6 | 9 | ExoVC acrylat2 | 1 | H1 | 3,7 | 565 | 41 |
| 7 | 7 | ExoVC acrylat2 | 3 | H1 | 4,5 | 521 | 13,6 |
| 8 | 5 | ExoVC acrylat2 | 5 | H1 | 5,2 | 236 | 6,1 |
| 9 | 3 | ExoVC acrylat2 | 7 | H1 | 5,9 | 187 | 4,1 |
| 10 | 9 | ExoVC | 1 | H1 | 4,0 | 236 | 25,2 |
| 11 | 10 | -- | 0 | H2 | - | 2540 | 40 |
| 12 | 10 | ExoVC acrylat1 | 0,5 | H2 | 2,4 | 3000 | 34,4 |
| 13 | 10 | ExoVC acrylat1 | 0,5 | H2 | 2,6 | 2413 | 30,6 |
| 14 | 10 | ExoVC acrylat1 | 0,5 | H2 | 2,7 | 2515 | 36,6 |
| 15 | 10 | ExoVC acrylat1 | 1 | H2 | 2,7 | 2657 | 13,4 |
| 16 | 10 | ExoVC | 1 | H2 | 2,5 | 1700 | 8,5 |

**Tabelle 2:**

| Tiefungsprüfung und Shore-Härte der gehärteten Epoxidharz-zusammensetzungen | | | | | | | | |
|---|---|---|---|---|---|---|---|---|
| Nr. | Epoxy verbindung | Verdünner | | Härter | | Tg | Tiefungs -prüfung | Shore-D härte |
| | Gew.-teile | Type | Gew.-Teile | Type | Gew. Teile | °C | mm | |
| 1 | 10 | -- | 0 | H1 | 3,4 | 92,6 | 0,6 | 89,5 |
| 2 | 9 | ExoVC acrylat1 | 1 | H1 | 4,0 | 69,9 | 9,0 | 89,0 |
| 3 | 7 | ExoVC acrylat1 | 3 | H1 | 5,3 | 41,8 | 9,0 | 86,6 |
| 4 | 5 | ExoVC acrylat1 | 5 | H1 | 6,8 | 24,7 | 9,0 | 76,9 |
| 5 | 3 | ExoVC acrylat1 | 7 | H1 | 8,0 | - | 9,0 | 36,9 |
| 6 | 9 | ExoVC acrylat2 | 1 | H1 | 3,7 | 74,8 | 0,7 | 88,4 |
| 7 | 7 | ExoVC acrylat2 | 3 | H1 | 4,5 | 53,7 | 0,9 | 87,6 |
| 8 | 5 | ExoVC acrylat2 | 5 | H1 | 5,2 | 33,8 | 9,0 | 86,5 |
| 9 | 3 | ExoVC acrylat2 | 7 | H1 | 5,9 | 22,5 | 9,0 | 77,7 |
| 10 | 9 | ExoVC | 1 | H1 | 4,0 | 62,5 | 0,5 | 88,2 |

## Patentansprüche

1. Verbindungen der Formel 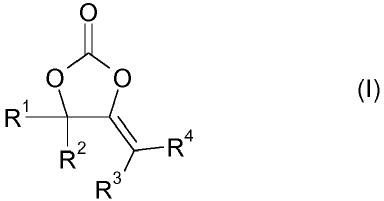 worin
R¹ für einen organischen Rest mit einer (Meth)acrylgruppe steht,
R², R³ und R⁴ unabhängig voneinander für ein H-Atom oder eine C1- bis C10-Alkylgruppe stehen.

2. Verbindungen gemäß Anspruch 1, **dadurch gekennzeichnet, dass**
R¹ für einen organischen Rest mit insgesamt maximal 24 C-Atomen steht und außer Sauerstoffatomen keine weiteren Heteroatome enthält.

3. Verbindungen gemäß Anspruch 1, **dadurch gekennzeichnet, dass**
R¹ eine Gruppe der Formel (II) 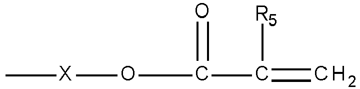 worin X für eine Bindung oder eine Alkylengruppe mit 1 bis 18 C-Atomen und R⁵ für ein H-Atom oder eine Methylgruppe steht,
oder eine Gruppe der Formel (III) 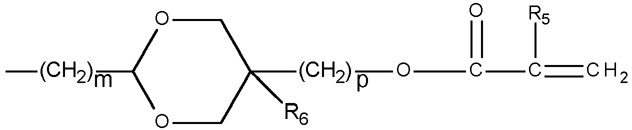 worin m und p unabhängig voneinander für 0 oder eine ganze Zahl von 1 bis 10 stehen, R⁵ die obige Bedeutung hat und R⁶ für ein H-Atom oder eine C1- bis C10-Alkylgruppe steht,
oder eine Gruppe der Formel (IV) 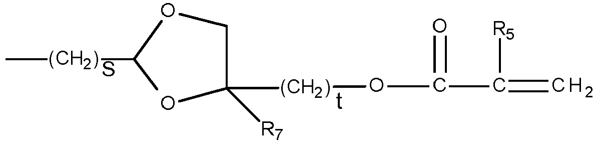 worin s und t unabhängig voneinander 0 oder eine ganze Zahl von 1 bis 10 stehen, R⁵ die obige Bedeutung hat und R⁷ für ein H-Atom oder eine C1- bis C10-Alkylgruppe steht, bedeutet.

4. Verbindungen gemäß einem der Ansprüche 1 bis 3, **dadurch gekennzeichnet, dass** R² für eine Methylgruppe und R³ und R⁴ für ein H-Atom stehen.

5. Verfahren zur Herstellung von Verbindungen der Formel (I) gemäß den Ansprüchen 1-4, in denen R¹ für eine Gruppe der Formel II gemäß Anspruch 3 steht, **dadurch kennzeichnet, dass**
- in einer ersten Stufe eine Verbindung mit einer endständigen Dreifachbindung mit einem Hydroxyalkanon oder Hydroxyalkanal umgesetzt wird, wobei sich die Dreifachbindung an die Carbonylgruppe des Hydroxyalkanon oder Hydroxyalkanal unter Ausbildung einer Dihydroxyverbindung addiert,
- in einer zweiten Stufe die Hydroxygruppe der erhaltenen Dihydroxyverbindung, welche nicht aus der Carbonylgruppe hervorgegangen ist, mit einer Schutzgruppe geschützt wird,
- in einer dritten Stufe mit Kohlendioxid der Ringschluss zur Carbonatgruppe erfolgt und
- in einer vierten Stufe die Schutzgruppe durch eine (Meth)acrylgruppe ersetzt wird.

6. Verfahren zur Herstellung von Verbindungen der Formel (I) in denen R¹ für eine Gruppe der Formel III steht, **dadurch kennzeichnet, dass**
- in einer ersten Stufe eine Verbindung mit einer endständigen Dreifachbindung mit einem Alkanon oder Alkanal, welches eine Acetalgruppe enthält, umgesetzt wird, wobei sich die Dreifachbindung an die Carbonylgruppe des Alkanon oder Alkanals unter Ausbildung einer Hydroxyverbindung addiert,
- in einer zweiten Stufe mit Kohlendioxid der Ringschluss zur Carbonatgruppe erfolgt,
- in einer dritten Stufe der Ringschluss zum 1,3-Dioxanring durch Umsetzung der Acetalgruppe mit einer Verbindung mit insgesamt mindestens drei Hydroxylgruppen wobei sich zwei der Hydroxylgruppen in 1,3 Stellung befinden, durchgeführt wird und
- in einer vierten Stufe die (Meth)acrylgruppe durch eine Veresterung oder Umesterung der verbleibenden Hydroxylgruppe eingeführt wird.

7. Verfahren zur Herstellung von Verbindungen der Formel (I) in denen R¹ für eine Gruppe der Formel IV steht, **dadurch kennzeichnet, dass**
- in einer ersten Stufe eine Verbindung mit einer endständigen Dreifachbindung mit einem Alkanon oder Alkanal, welches eine Acetalgruppe enthält, umgesetzt wird, wobei sich die Dreifachbindung an die Carbonylgruppe des Alkanon oder Alkanals unter Ausbildung einer Hydroxyverbindung addiert,
- in einer zweiten Stufe mit Kohlendioxid der Ringschluss zur Carbonatgruppe erfolgt,
- in einer dritten Stufe der Ringschluss zum 1,3-Dioxolanring durch Umsetzung der Acetalgruppe mit einer Verbindung mit insgesamt mindestens drei Hydroxylgruppen wobei sich zwei der Hydroxylgruppen in 1,2 Stellung befinden, durchgeführt wird und
- in einer vierten Stufe die (Meth)acrylgruppe durch eine Veresterung oder Umesterung der verbleibenden Hydroxylgruppe eingeführt wird.

8. Verwendung von Verbindungen der Formel I in Epoxidharz-zusammensetzungen

9. Epoxidharz-zusammensetzungen, enthaltend
a) Verbindungen der Formel (I) und
b) Verbindungen mit mindestens einer Epoxygruppe (kurz Epoxyverbindungen) und
c) gegebenenfalls Härter für Epoxyverbindungen und
d) gegebenenfalls weitere Bestandteile

10. Epoxidharz-zusammensetzungen gemäß Anspruch 9, **dadurch gekennzeichnet, dass** sie 0,1 bis 10000 Gewichtsteile der Verbindung der Formel I auf 100 Gewichtsteile Epoxyverbindungen enthalten.

11. Epoxidharz-zusammensetzungen gemäß Anspruch 9 oder 10, **dadurch gekennzeichnet, dass** es sich bei den Härtern um Verbindungen mit mindestens einer primären oder sekundären Aminogruppe (kurz Amino-Härter) handelt

12. Verwendung von Epoxidharzzusammensetzungen gemäß einem der Ansprüche 9 bis 11 als zweikomponentiges Bindemittelsystem (2K-Bindemittelsystem) aus einer Komponente A, enthaltend
a) Verbindungen der Formel (I) und
b) Verbindungen mit mindestens einer Epoxygruppe (kurz Epoxyverbindungen) und einer Komponenten B, enthaltend
c) Härter für Epoxyverbindungen

13. Verwendung der Epoxyharz-zusammensetzungen gemäß einem der Ansprüche 9-11 als Beschichtungsmassen, Gießmassen, zur Herstellung von Verbundwerkstoffen, als Klebstoffe oder zur Imprägnierung von Fasern oder Fasergeweben

## Claims

1. A compound of the formula 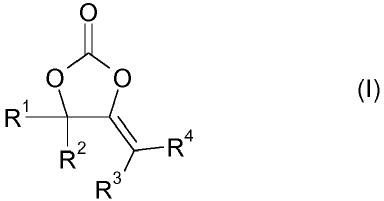 in which
R¹ is an organic radical having a (meth)acryloyl group and
R², R³, and R⁴ independently of one another are an H atom or a C1 to C10 alkyl group.

2. The compound according to claim 1, wherein
R¹ is an organic radical having a total of not more than 24 C atoms and comprising no heteroatoms other than oxygen atoms.

3. The compound according to claim 1, wherein
R¹ is a group of the formula (II) 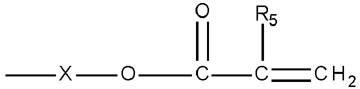 in which X is a bond or an alkylene group having 1 to 18 C atoms and R⁵ is an H atom or a methyl group,
or a group of the formula (III) 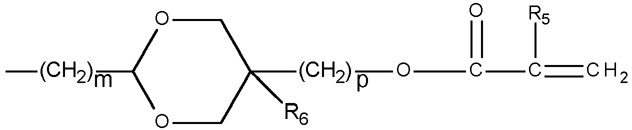 in which m and p independently of one another are 0 or an integer from 1 to 10, R⁵ has the above definition, and R⁶ is an H atom or a C1 to C10 alkyl group,
or a group of the formula (IV) 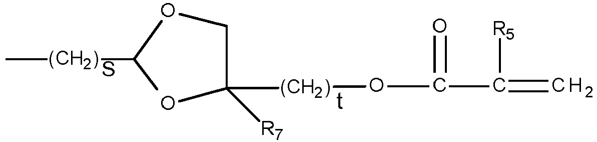 in which s and t independently of one another are 0 or an integer from 1 to 10, R⁵ has the above definition, and R⁷ is an H atom or a C1 to C10 alkyl group.

4. The compound according to any of claims 1 to 3, wherein R² is a methyl group and R³ and R⁴ are an H atom.

5. A process for preparing a compound of the formula (I) according to claims 1-4, in which R¹ is a group of the formula II according to claim 3, wherein
- in a first stage, a compound having a terminal triple bond is reacted with a hydroxyalkanone or hydroxyalkanal, the triple bond undergoing addition to the carbonyl group of the hydroxyalkanone or hydroxyalkanal to form a dihydroxy compound,
- in a second stage, the hydroxyl group of the resulting dihydroxy compound that did not originate from the carbonyl group is protected with a protecting group,
- in a third stage, the ring closure to form the carbonate group takes place with carbon dioxide, and
- in a fourth stage, the protecting group is replaced by a (meth)acryloyl group.

6. A process for preparing a compound of the formula (I) in which R¹ is a group of the formula III, wherein
- in a first stage, a compound having a terminal triple bond is reacted with an alkanone or alkanal which comprises an acetal group, the triple bond undergoing addition to the carbonyl group of the alkanone or alkanal to form a hydroxy compound,
- in a second stage, the ring closure to form the carbonate group takes place with carbon dioxide,
- in a third stage, the ring closure to form the 1,3-dioxane ring is carried out by reaction of the acetal group with a compound having a total of at least three hydroxyl groups, with two of the hydroxyl groups being located in 1,3 position, and
- in a fourth stage, the (meth)acryloyl group is introduced by esterification or transesterification of the remaining hydroxyl group.

7. A process for preparing a compound of the formula (I) in which R¹ is a group of the formula IV, wherein
- in a first stage, a compound having a terminal triple bond is reacted with an alkanone or alkanal which comprises an acetal group, the triple bond undergoing addition to the carbonyl group of the alkanone or alkanal to form a hydroxy compound,
- in a second stage, the ring closure to form the carbonate group takes place with carbon dioxide,
- in a third stage, the ring closure to form the 1,3-dioxolane ring is carried out by reaction of the acetal group with a compound having a total of at least three hydroxyl groups, with two of the hydroxyl groups being located in 1,2 position, and
- in a fourth stage, the (meth)acryloyl group is introduced by esterification or transesterification of the remaining hydroxyl group.

8. The use of a compound of the formula I in an epoxy resin composition.

9. An epoxy resin composition comprising
a) compounds of the formula (I) and
b) compounds having at least one epoxy group (epoxy compounds for short), and
c) optionally hardeners for epoxy compounds, and
d) optionally further constituents.

10. The epoxy resin composition according to claim 9, comprising 0.1 to 10 000 parts by weight of the compound of the formula I per 100 parts by weight of epoxy compounds.

11. The epoxy resin composition according to claim 9 or 10, wherein the hardeners are compounds having at least one primary or secondary amino group (amino hardeners for short).

12. The use of an epoxy resin composition according to any of claims 9 to 11 as a two-component binder system (2K binder system) composed of a component A comprising
a) compounds of the formula (I) and
b) compounds having at least one epoxy group (epoxy compounds for short), and
a component B comprising
c) hardeners for epoxy compounds.

13. The use of the epoxy resin compositions according to any of claims 9-11 as coating material, casting material, for producing composite materials, as adhesive, or for impregnating fibers or woven fiber fabrics.

## Revendications

1. Composés de formule 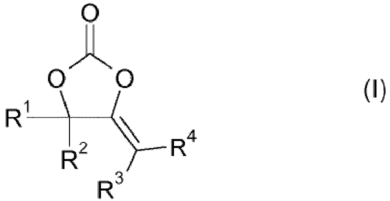 dans laquelle
R¹ représente un radical organique contenant un groupe (méth)acrylique,
R², R³ et R⁴ représentent indépendamment les uns des autres un atome H ou un groupe alkyle en C1 à C10.

2. Composés selon la revendication 1, **caractérisés en ce que**
R¹ représente un radical organique contenant au total au plus 24 atomes C et ne contenant pas d'autres hétéroatomes que des atomes d'oxygène.

3. Composés selon la revendication 1, **caractérisés en ce que**
R¹ représente un groupe de formule (II) 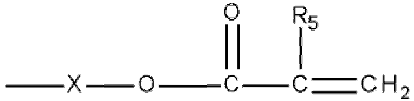 dans laquelle X représente une liaison ou un groupe alkylène de 1 à 18 atomes C, et R⁵ représente un atome H ou un groupe méthyle,
ou un groupe de formule (III) 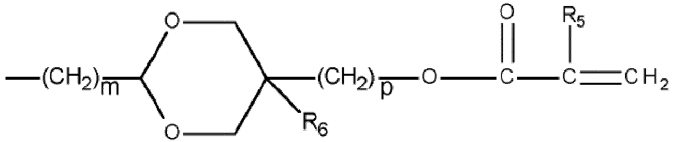 dans laquelle m et p représentent indépendamment l'un de l'autre 0 ou un nombre entier de 1 à 10,
R⁵ a la signification ci-dessus, et R⁶ représente un atome H ou un groupe alkyle en C1 à C10,
ou un groupe de formule (IV) 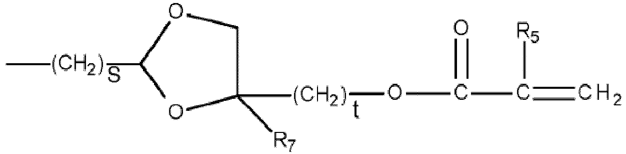 dans laquelle s et t représentent indépendamment l'un de l'autre 0 ou un nombre entier de 1 à 10, R⁵ a la signification ci-dessus, et R⁷ représente un atome H ou un groupe alkyle en C1 à C10.

4. Composés selon l'une quelconque des revendications 1 à 3, **caractérisés en ce que** R² représente un groupe méthyle, et R³ et R⁴ représentent un atome H.

5. Procédé de fabrication de composés de formule (I) selon les revendications 1 à 4, dans lesquels R¹ représente un groupe de formule II selon la revendication 3, **caractérisé en ce que**
- lors d'une première étape, un composé contenant une triple liaison terminale est mis en réaction avec une hydroxyalcanone ou un hydroxyalcanal, la triple liaison s'additionnant sur le groupe carbonyle de l'hydroxyalcanone ou de l'hydroxyalcanal avec formation d'un composé dihydroxy,
- lors d'une deuxième étape, le groupe hydroxy du composé dihydroxy obtenu qui ne provient pas du groupe carbonyle est protégé avec un groupe protecteur,
- lors d'une troisième étape, la fermeture de cycle pour former le groupe carbonate a lieu avec du dioxyde de carbone, et
- lors d'une quatrième étape, le groupe protecteur est remplacé par un groupe (méth)acrylique.

6. Procédé de fabrication de composés de formule (I) dans lesquels R¹ représente un groupe de formule III, **caractérisé en ce que**
- lors d'une première étape, un composé contenant une triple liaison terminale est mis en réaction avec une alcanone ou un alcanal qui contient un groupe acétal, la triple liaison s'additionnant sur le groupe carbonyle de l'alcanone ou de l'alcanal avec formation d'un composé hydroxy,
- lors d'une deuxième étape, la fermeture de cycle pour former le groupe carbonate a lieu avec du dioxyde de carbone,
- lors d'une troisième étape, la fermeture de cycle pour former le cycle 1,3-dioxane est réalisée par mise en réaction du groupe acétal avec un composé contenant au total au moins trois groupes hydroxyle, deux des groupes hydroxyle se trouvant en position 1,3, et
- lors d'une quatrième étape, le groupe (méth)acrylique est introduit par une estérification ou transestérification du groupe hydroxyle restant.

7. Procédé de fabrication de composés de formule (I) dans lesquels R¹ représente un groupe de formule IV, **caractérisé en ce que**
- lors d'une première étape, un composé contenant une triple liaison terminale est mis en réaction avec une alcanone ou un alcanal qui contient un groupe acétal, la triple liaison s'additionnant sur le groupe carbonyle de l'alcanone ou de l'alcanal avec formation d'un composé hydroxy,
- lors d'une deuxième étape, la fermeture de cycle pour former le groupe carbonate a lieu avec du dioxyde de carbone,
- lors d'une troisième étape, la fermeture de cycle pour former le cycle 1,3-dioxane est réalisée par mise en réaction du groupe acétal avec un composé contenant au total au moins trois groupes hydroxyle, deux des groupes hydroxyle se trouvant en position 1,2, et
- lors d'une quatrième étape, le groupe (méth)acrylique est introduit par une estérification ou transestérification du groupe hydroxyle restant.

8. Utilisation de composés de formule I dans des compositions de résine époxyde.

9. Compositions de résine époxyde, contenant :
a) des composés de formule (I) et
b) des composés contenant au moins un groupe époxy (en abrégé composés époxy) et
c) éventuellement des agents de durcissement pour composés époxy, et
d) éventuellement d'autres constituants.

10. Compositions de résine époxy selon la revendication 9, **caractérisées en ce qu'**elles contiennent 0,1 à 10 000 parties en poids du composé de formule I pour 100 parties en poids de composés époxy.

11. Compositions de résine époxy selon la revendication 9 ou 10, **caractérisées en ce que** les agents de durcissement consistent en des composés contenant au moins un groupe amino primaire ou secondaire (en abrégé agents de durcissement amino).

12. Utilisation de compositions de résine époxyde selon l'une quelconque des revendications 9 à 11 en tant que système liant bicomposant (système liant 2K) constitué par un composant A, contenant :
a) des composés de formule (I) et
b) des composés contenant au moins un groupe époxy (en abrégé composés époxy) et
un composant B, contenant :
c) des agents de durcissement pour composés époxy.

13. Utilisation de compositions de résine époxy selon l'une quelconque des revendications 9 à 11 en tant que matériaux de revêtement, matériaux à couler, pour la fabrication de matériaux composites, en tant qu'adhésifs ou pour l'imprégnation de fibres ou de tissus fibreux.
